# EUROPÄISCHE PATENTSCHRIFT

(11) **EP 2 265 689 B1**
(45) Veröffentlichungstag und Bekanntmachung des Hinweises auf die Patenterteilung: **20.06.2018**
(21) Anmeldenummer: 09732312.5
(22) Anmeldetag: 17.03.2009
(51) Int. Cl.: C09K 11/06, H01L 51/50

(54) **NEUE MATERIALIEN FÜR ORGANISCHE ELEKTROLUMINESZENZVORRICHTUNGEN**
NOVEL MATERIALS FOR ORGANIC ELECTROLUMINESCENCE DEVICES
NOUVEAUX MATÉRIAUX POUR DES DISPOSITIFS ÉLECTROLUMINESCENTS ORGANIQUES

(30) Priorität: 14.04.2008 DE 102008018670
(43) Veröffentlichungstag der Anmeldung: 29.12.2010
(73) Patentinhaber: Merck Patent GmbH, 64293 Darmstadt (DE)
(72) Erfinder: BUESING, Arne, 65929 Frankfurt am Main (DE); HEIL, Holger, 60389 Frankfurt (DE); STOESSEL, Philipp, 60487 Frankfurt am Main (DE)
(86) Internationale Anmeldenummer: PCT/EP2009/001938
(87) Internationale Veröffentlichungsnummer: WO 2009/127307

(56) Entgegenhaltungen:
- DE-A1-102004 031 000
- DE-A1-102005 058 557
- DE-A1-102006 031 990
- DE-A1-102006 035 035
- US-A1- 2002 132 134
- US-A1- 2004 131 881

## Beschreibung

Die vorliegende Erfindung betrifft organische Halbleiter und deren Verwendung in organischen elektronischen Vorrichtungen.

Organische Halbleiter werden für eine Reihe verschiedenartiger elektronischer Anwendungen entwickelt. Der Aufbau organischer Elektrolumineszenzvorrichtungen (OLEDs), in denen diese organischen Halbleiter als funktionelle Materialien eingesetzt werden, ist beispielsweise in US 4539507, US 5151629, EP 0676461 und WO 98/27136 beschrieben. Allerdings sind noch weitere Verbesserungen wünschenswert, um diese Vorrichtungen für hochwertige und langlebige Displays verwenden zu können. So stellen insbesondere die Lebensdauer und die Effizienz blau emittierender organischer Elektrolumineszenzvorrichtungen derzeit noch ein Problem dar, bei welchem es noch Verbesserungsbedarf gibt. Weiterhin ist es erforderlich, dass die Verbindungen eine hohe thermische Stabilität und eine hohe Glasübergangstemperatur aufweisen und sich unzersetzt sublimieren lassen. Insbesondere für Anwendung bei erhöhter Temperatur ist eine hohe Glasübergangstemperatur für die Erreichung hoher Lebensdauern essentiell.

Für fluoreszierende OLEDs werden gemäß dem Stand der Technik vor allem kondensierte Aromaten, insbesondere Anthracenderivate, als Host-materialien vor allem für blau emittierende Elektrolumineszenzvorrichtungen verwendet, z. B. 9,10-Bis(2-naphthyl)anthracen (US 5935721). In WO 03/095445 und in CN 1362464 werden 9,10-Bis(1-naphthyl)-anthracen-Derivate für die Verwendung in OLEDs offenbart. Weitere Anthracenderivate sind in WO 01/076323, in WO 01/021729, in WO 04/013073, in WO 04/018588, in WO 03/087023 oder in WO 04/018587 offenbart. Hostmaterialien, basierend auf Arylsubstituierten Pyrenen und Chrysenen, werden in WO 04/016575 offenbart, Hostmaterialien basierend auf Benzanthracenen in WO 08/145239. In US 2004/131881 werden Fluorenederivate enthaltend mindestens ein kondensiertes Aromat für die Verwendung in OLEDs offenbart. Es ist für hochwertige Anwendungen wünschenswert, verbesserte Hostmaterialien zur Verfügung zu haben. Dasselbe gilt auch für Hostmaterialien für grün und rot fluoreszierende Dotanden.

Als Stand der Technik bei blau emittierenden Verbindungen kann die Verwendung von Arylvinylaminen genannt werden (z. B. WO 04/013073, WO 04/016575, WO 04/018587). Diese Verbindungen sind jedoch thermisch instabil und lassen sich nicht unzersetzt verdampfen, was einen hohen technischen Aufwand für die OLED-Herstellung erfordert und somit einen technischen Nachteil darstellt. Daher ist es für hochwertige Anwendungen notwendig, verbesserte Emitter besonders im Bezug auf Device- und Sublimationsstabilität sowie Emissionsfarbe zur Verfügung zu haben. Weiterhin wäre es vorteilhaft, Emitter zur Verfügung zu haben, welche ein schmaleres Emissionsspektrum aufweisen.

Es besteht also weiterhin Bedarf an verbesserten Materialien, insbesondere Host-Materialien für fluoreszierende Emitter, vor allem für grün und rot fluoreszierende Emitter, aber auch für blau fluoreszierende Emitter, und fluoreszierenden Materialien, die thermisch stabil sind, die in organischen elektronischen Vorrichtungen zu guten Effizienzen und gleichzeitig zu hohen Lebensdauern führen, die bei der Herstellung und beim Betrieb der Vorrichtung zu reproduzierbaren Ergebnissen führen und die synthetisch einfach zugänglich sind. Auch bei Loch- und Elektronentransportmaterialien sind weitere Verbesserungen erforderlich.

Überraschend wurde gefunden, dass Anthracenderivate, welche in 9- oder 9,10-Position substituiert sind und an welche in 1,2-Position oder 2,3-Position oder 3,4-Position und/oder in 5,6-Position oder 6,7-Position oder 7,8-Position eine Indenogruppe ankondensiert ist, sich sehr gut für die Verwendung in organischen Elektrolumineszenzvorrichtungen eignen. Dies gilt ebenso, wenn statt der Indenogruppe entsprechende heterocyclische Gruppen, wie zum Beispiel Indologruppen oder Benzothienylgruppen, ankondensiert sind. Mit diesen Verbindungen ist eine Steigerung der Effizienz und vor allem der Lebensdauer der organischen elektronischen Vorrichtung im Vergleich zu Materialien gemäß dem Stand der Technik möglich. Dies trifft insbesondere auf blau fluoreszierende Vorrichtungen zu. Weiterhin weisen diese Verbindungen eine hohe thermische Stabilität auf. Generell sind diese Materialien sehr gut für die Verwendung in organischen elektronischen Vorrichtungen geeignet, da sie eine hohe Glasübergangstemperatur aufweisen. Diese Materialien und deren Verwendung in organischen elektronischen Vorrichtungen sind daher der Gegenstand der vorliegenden Erfindung.

Als nächstliegender Stand der Technik kann die US 2002/132134 betrachtet werden. Darin werden kondensierte Aromaten offenbart, an welche zwei Aryl-substituierte Indenogruppen ankondensiert sind. Diese Verbindungen weisen jedoch einen großen Stokes-Shift auf, welcher möglicherweise verursacht ist durch die Rotationsfreiheitsgrade der Arylsubstituenten. Daher eignen sich die in US 2002/132134 offenbarten Verbindungen nicht als Hostmaterial für tiefblaue Emitter. Hier besteht daher noch Verbesserungsbedarf. Weiterhin wäre es vorteilhaft, Verbindungen zur Verfügung zu haben, welche ein engeres Emissionsspektrum aufweisen.

Weiterhin ist als Emitter 9,10-Diphenylanthracen bekannt. Dieses weist zwar 100 % Fluoreszenzquanteneffizienz auf (H. Du et al., Photochemistry and Photobiology 1998, 68, 141-142), jedoch liegt die Emission zu weit im blauen Bereich, so dass diese Verbindung nicht als blauer Emitter verwendet werden kann.

Der Übersichtlichkeit halber werden im Folgenden die Struktur und die Nummerierung des Anthracens dargestellt:

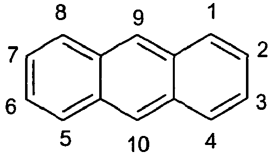

Gegenstand der Erfindung sind daher Verbindungen gemäß der Formel (1) und (2), 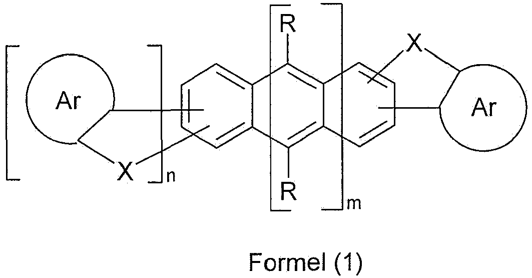 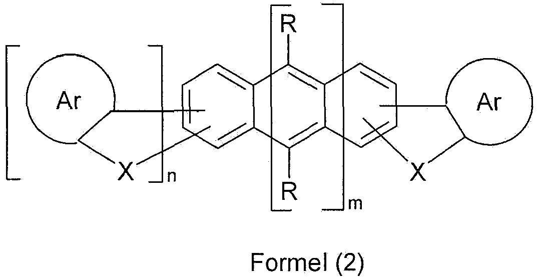 dabei können in der Anthraceneinheit auch ein oder mehrere unsubstituierte Kohlenstoffatome auch durch Stickstoff ersetzt sein; weiterhin gilt für die verwendeten Symbole und Indizes:
- X: ist bei jedem Auftreten gleich oder verschieden eine bivalente Brücke, ausgewählt aus C(R¹)₂;
- Ar: ist bei jedem Auftreten gleich oder verschieden eine Arylgruppe mit 6 bis 10 C-Atomen oder eine Heteroarylgruppe mit 4 bis 9 C-Atomen;
- R¹: ist bei jedem Auftreten gleich oder verschieden methyl, ethyl, iso-Propyl, tert-Butyl, wobei ein H-Atom oder mehrere H-Atome durch F ersetzt sein können, oder eine Arylgruppe mit 6 bis 14 C-Atomen, die jeweils durch einen oder mehrere Reste R² substituiert sein kann; dabei können zwei oder mehrere benachbarte Substituenten R¹ auch miteinander ein mono- oder polycyclisches, aliphatisches oder aromatisches Ringsystem bilden;
- R: ist bei jedem Auftreten gleich oder verschieden ein aromatisches oder heteroaromatisches Ringsystem mit 5 bis 14 aromatischen Ringatomen, das jeweils durch einen oder mehrere Reste R² substituiert sein kann;
- R²: ist bei jedem Auftreten gleich oder verschieden H, D, F, Cl, Methyl, Ethyl, n-Propyl, i-Propyl, n-Butyl, i-Butyl, s-Butyl, t-Butyl, wobei ein oder mehrere H-Atome durch F, Cl, oder CN ersetzt sein können;
- m: ist 1;
- n: ist 0 oder 1;
dadurch gekennzeichnet, dass die Gruppen Ar unsubstituiert sind.

In den Strukturen der Formel (1) und (2) bindet die Gruppe Ar über ein Kohlenstoffatom an das Anthracen und die Gruppe X bindet an ein benachbartes Kohlenstoffatom der Gruppe Ar und an ein benachbartes Kohlenstoffatom des Anthracens. Analog zum Indenofluoren sind hier für n = 1 auch cis- und trans-Diindenoanthracenderivate und entsprechende Derivate mit anderen ankondensierten Gruppen möglich, wobei Strukturen der Formel (1) zu trans-Derivaten und Strukturen der Formel (2) zu cis-Derivaten führen.

Bevorzugt weisen die Verbindungen gemäß Formel (1) eine Glasübergangstemperatur T_{G} von größer als 70 °C auf, besonders bevorzugt größer als 100 °C, ganz besonders bevorzugt größer als 130 °C.

Eine Arylgruppe im Sinne dieser Erfindung enthält 6 bis 40 C-Atome; eine Heteroarylgruppe im Sinne dieser Erfindung enthält 2 bis 40 C-Atome und mindestens 1 Heteroatom, mit der Maßgabe, dass die Summe aus C-Atomen und Heteroatomen mindestens 5 ergibt. Die Heteroatome sind bevorzugt ausgewählt aus N, O und/oder S. Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin, Thiophen, etc., oder eine kondensierte Aryl- oder Heteroarylgruppe, beispielsweise Naphthalin, Anthracen, Pyren, Chinolin, Isochinolin, etc., verstanden.

Ein aromatisches Ringsystem im Sinne dieser Erfindung enthält 6 bis 40 C-Atome im Ringsystem. Ein heteroaromatisches Ringsystem im Sinne dieser Erfindung enthält 2 bis 40 C-Atome und mindestens ein Heteroatom im Ringsystem, mit der Maßgabe, dass die Summe aus C-Atomen und Heteroatomen mindestens 5 ergibt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine kurze, nicht-aromatische Einheit (bevorzugt weniger als 10 % der von H verschiedenen Atome), wie z. B. ein sp³-hybridisiertes C-, N- oder O-Atom, unterbrochen sein können. So sollen beispielsweise auch Systeme wie 9,9'-Spirobifluoren, 9,9-Diarylfluoren, Triarylamin, Diarylether, Stilben, Benzophenon, etc. als aromatische Ringsysteme im Sinne dieser Erfindung verstanden werden. Ebenso werden unter einem aromatischen bzw. heteroaromatischen Ringsystem Systeme verstanden, in denen mehrere Aryl- bzw. Heteroarylgruppen durch Einfachbindungen miteinander verknüpft sind, beispielsweise Biphenyl, Terphenyl oder Bipyridin.

Im Rahmen der vorliegenden Erfindung werden unter einer C₁- bis C₄₀-Alkylgruppe, in der auch einzelne H-Atome oder CH₂-Gruppen durch die oben genannten Gruppen substituiert sein können, besonders bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, Cyclopentyl, n-Hexyl, Cyclohexyl, n-Heptyl, Cycloheptyl, n-Octyl, Cyclooctyl, 2-Ethylhexyl, Trifluormethyl, Pentafluorethyl oder 2,2,2-Trifluorethyl verstanden. Unter einer Alkenylgruppe werden im Sinne dieser Erfindung bevorzugt die Reste Ethenyl, Propenyl, Butenyl, Pentenyl, Cyclopentenyl, Hexenyl, Cyclohexenyl, Heptenyl, Cycloheptenyl, Octenyl oder Cyclooctenyl verstanden. Unter einer Alkinylgruppe werden im Sinne dieser Erfindung bevorzugt Ethinyl, Propinyl, Butinyl, Pentinyl, Hexinyl, Heptinyl oder Octinyl verstanden. Unter einer C₁- bis C₄₀-Alkoxygruppe werden besonders bevorzugt Methoxy, Trifluormethoxy, Ethoxy, n-Propoxy, i-Propoxy, n-Butoxy, i-Butoxy, s-Butoxy, t-Butoxy oder 2-Methylbutoxy verstanden. Unter einem aromatischen oder heteroaromatischen Ringsystem mit 5 - 40 aromatischen Ringatomen, welches noch jeweils mit den oben genannten Resten R substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Phenanthren, Benzanthracen, Pyren, Chrysen, Perylen, Fluoranthen, Naphthacen, Pentacen, Benzpyren, Biphenyl, Biphenylen, Terphenyl, Terphenylen, Fluoren, Spirobifluoren, Dihydrophenanthren, Dihydropyren, Tetrahydropyren, cis- oder trans-Indenofluoren, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Eine bevorzugte Ausführungsform der Verbindungen gemäß Formel (1) und (2) sind die Verbindungen der Formeln (3) bis (15), 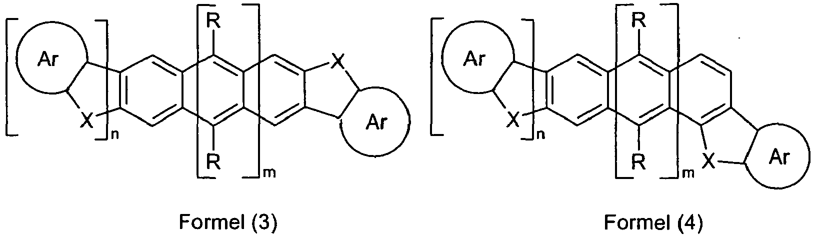 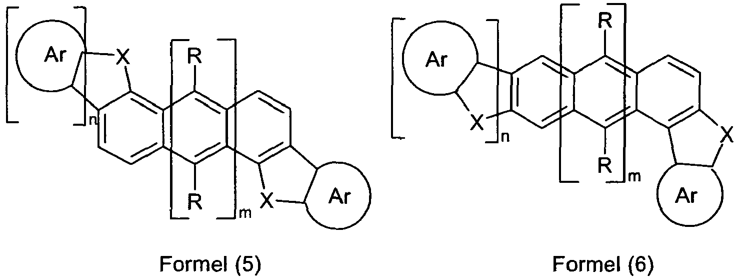 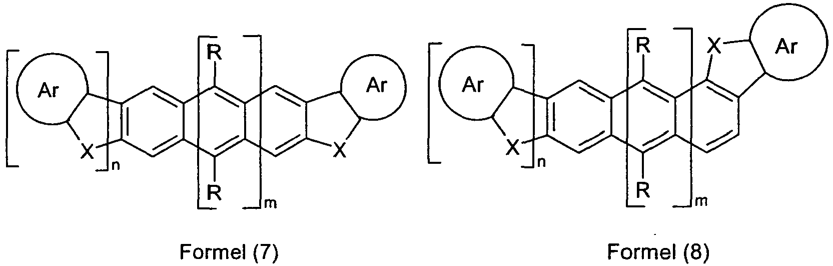 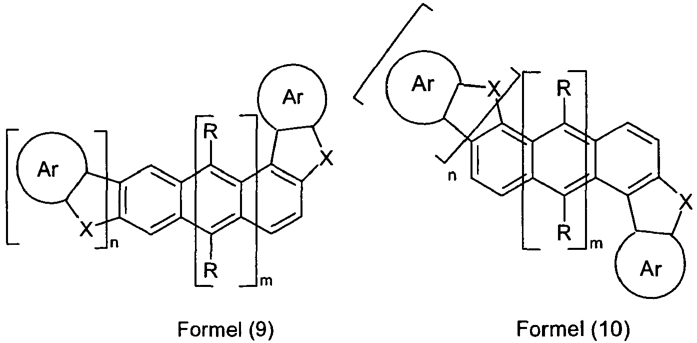 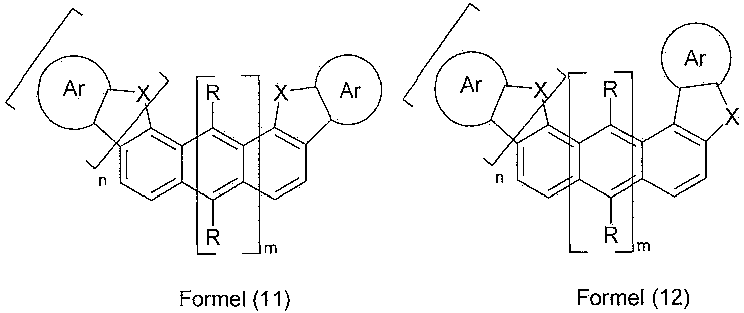 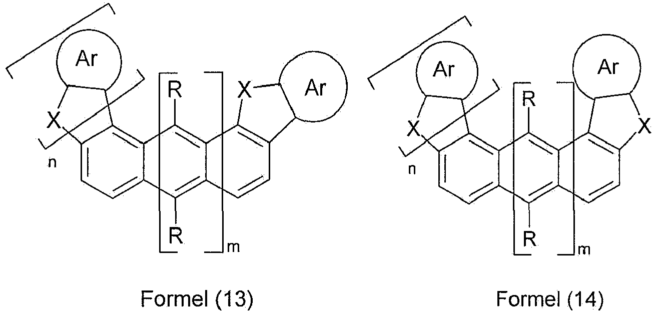 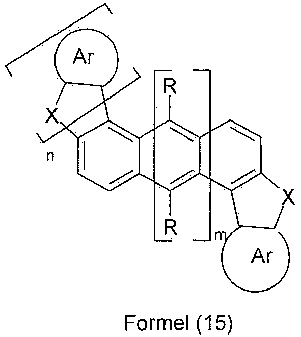 wobei in der Anthraceneinheit auch ein oder mehrere unsubstituierte Kohlenstoffatome durch Stickstoff ersetzt sein können und wobei die Symbole und Indizes die oben aufgeführten Bedeutungen haben.

In einer bevorzugten Ausführungsform der Erfindung steht das Symbol Ar gleich oder verschieden bei jedem Auftreten für Benzol, Naphthalin, Thiophen, Pyrrol, Furan, Pyridin, Pyrimidin, Pyrazin, Pyridazin, Chinolin, Chinoxalin, Triazin, Triazol, Imidazol, Benzimidazol, Benzothiophen, Indol und Benzofuran, insbesondere für Benzol, Naphthalin oder Thiophen.

In einer bevorzugten Ausführungsform der Erfindung sind die beiden Gruppen Ar für n = 1 gleich gewählt. Dies begründet sich mit der besseren synthetischen Zugänglichkeit der Verbindungen.

Von den Verbindungen der Formeln (3) bis (15) sind insbesondere die Verbindungen der Formeln (3), (4) und (5) bevorzugt, da diese ausgehend von synthetisch leicht zugänglichem 2-Bromanthrachinon für n = 1 synthetisiert werden können.

Eine besonders bevorzugte Ausführungsform der Verbindungen gemäß den Formeln (3) bis (15) sind die Verbindungen der Formeln (3a) bis (15a), (3b) bis (15b) und (3c) bis (15c), 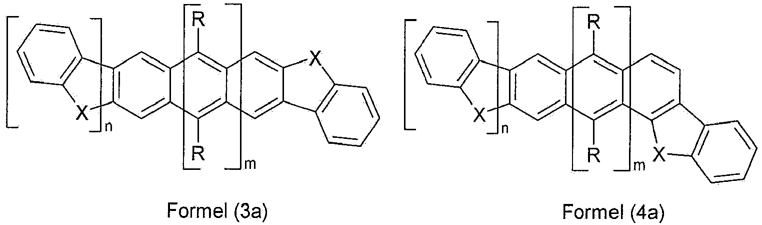 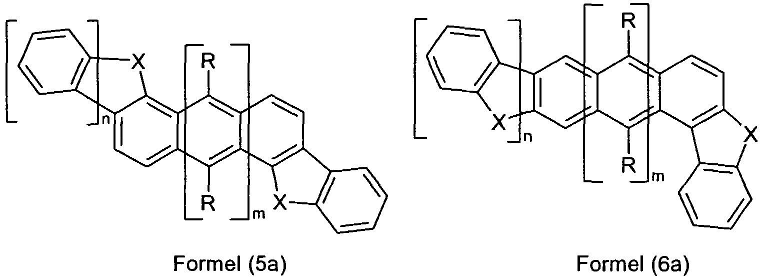 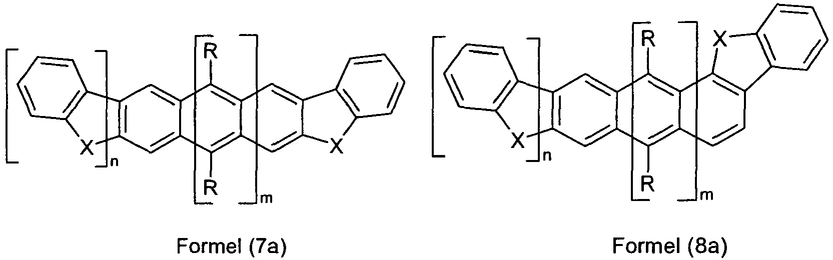 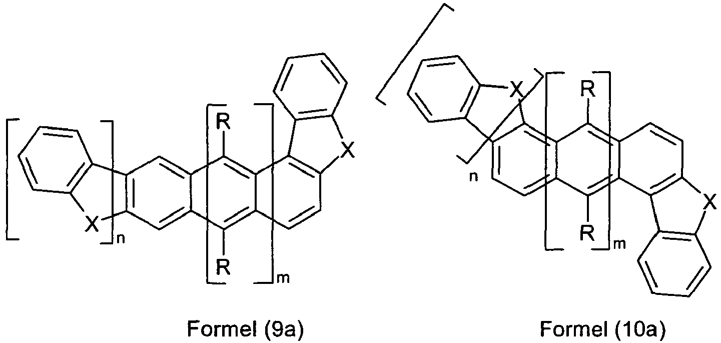 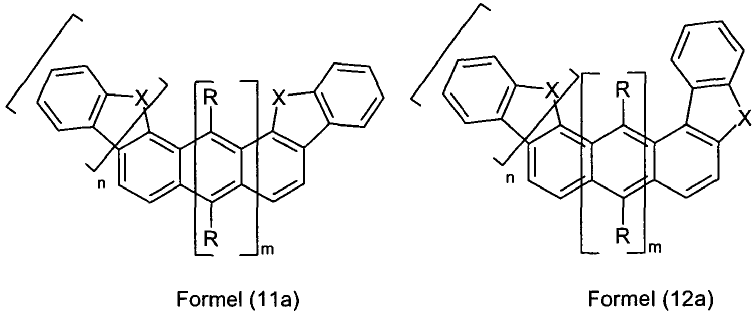 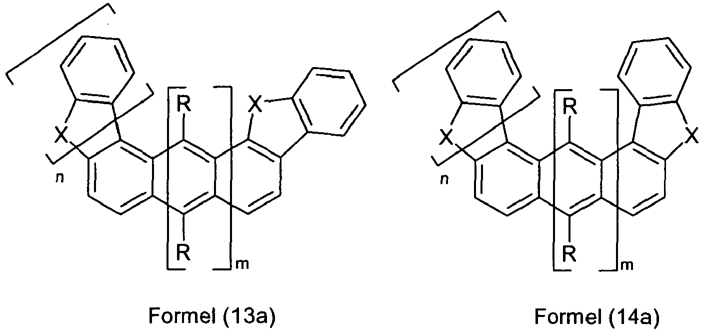 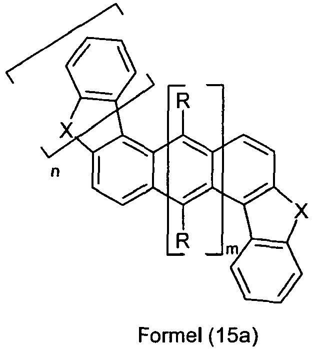 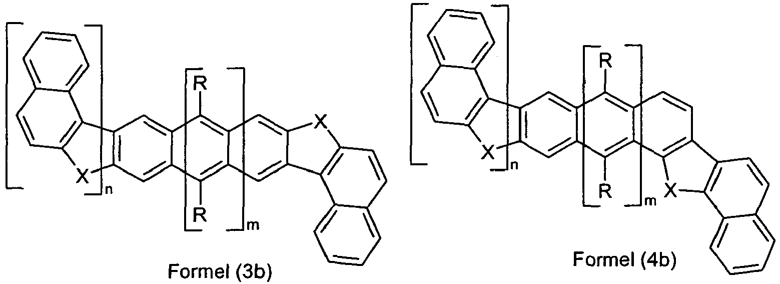 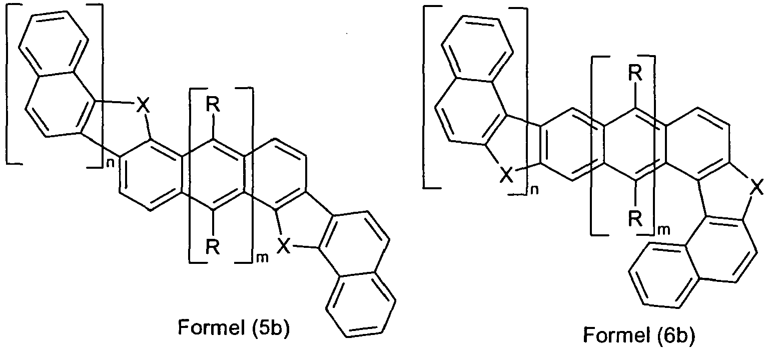 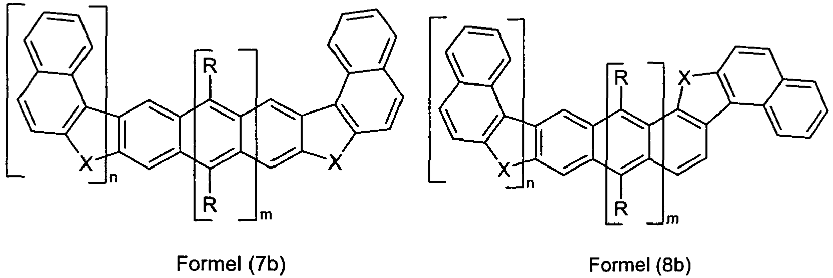 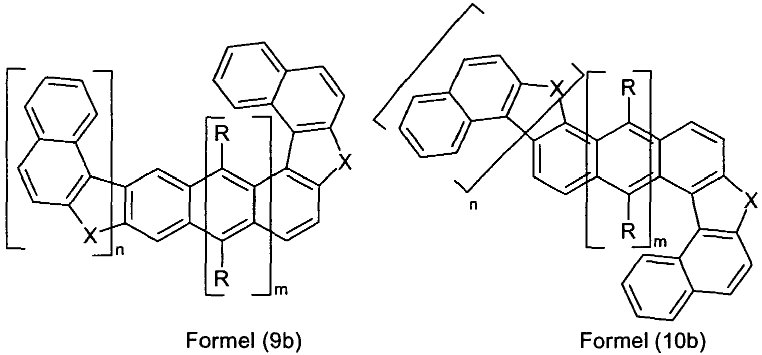 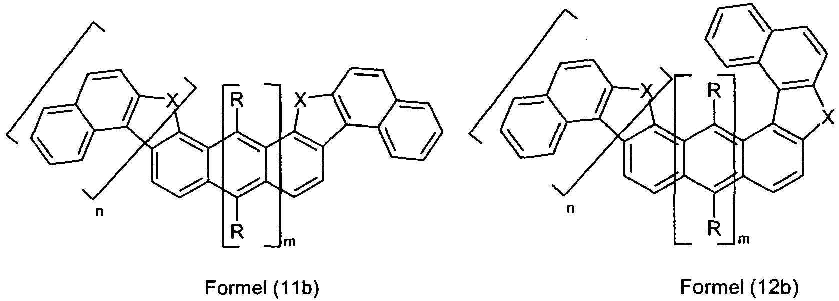 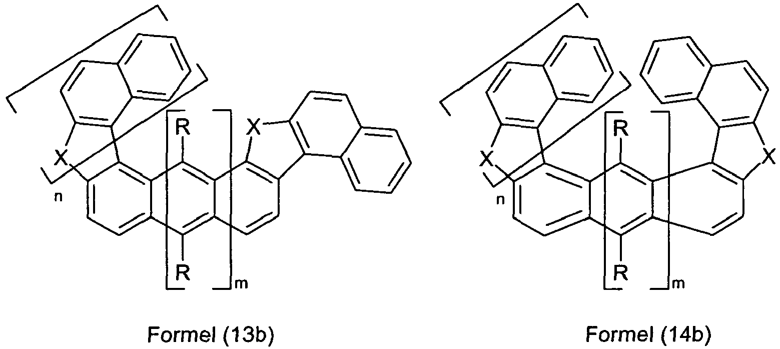 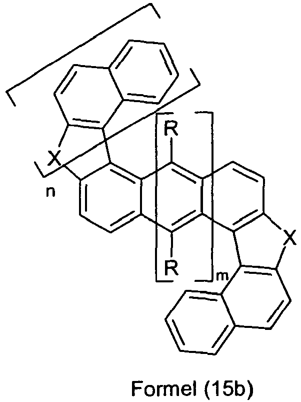 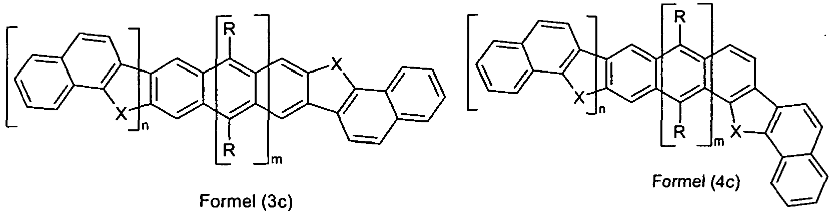 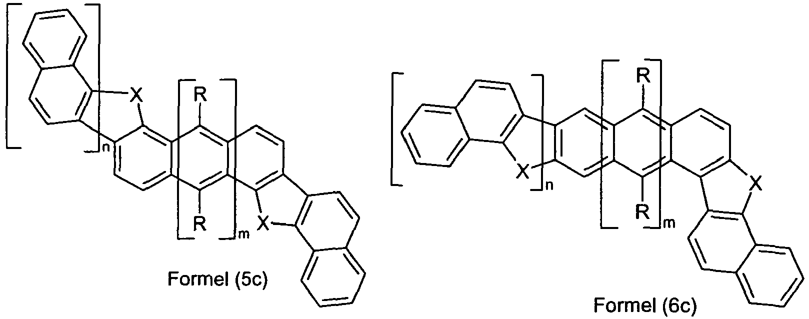 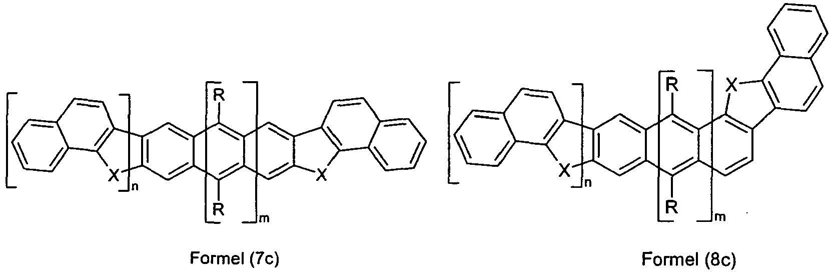 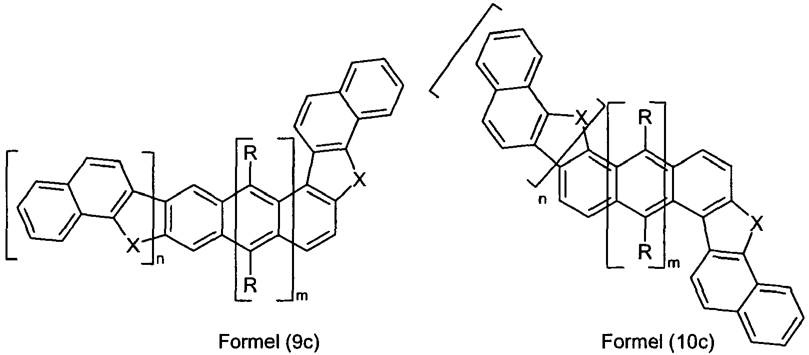 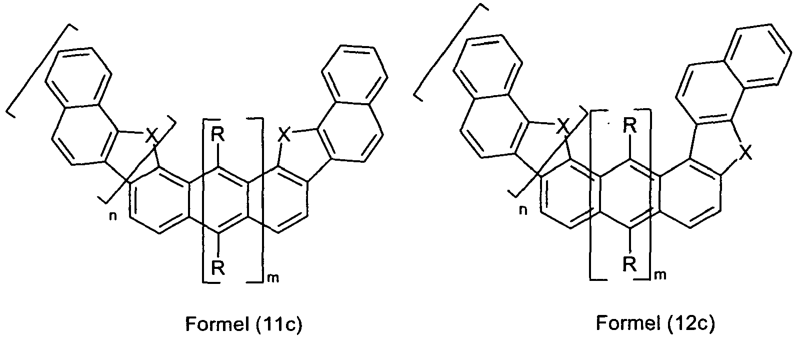 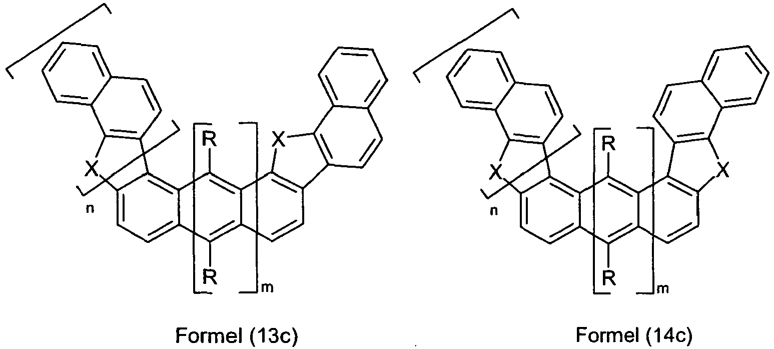 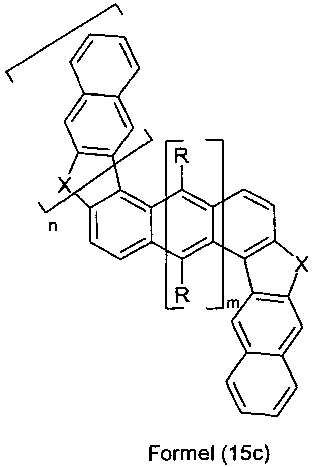 wobei die Symbole und Indizes die oben aufgeführten Bedeutungen haben. Besonders bevorzugt sind die oben abgebildeten Verbindungen der Formel (3a) bis (15a), insbesondere die Verbindungen der Formel (3a), (4a) und (5a).

Der Index m = 1, d. h. es handelt sich bei der zentralen Einheit um ein Anthracen.

Die Gruppe X steht für C(R¹)₂. Dabei sei hier nochmals explizit darauf hingewiesen, dass hier für X = C(R¹)₂ die Reste R¹ auch miteinander ein aromatisches oder aliphatisches Ringsystem bilden können. Wenn mehrere Reste R¹ an einer Gruppe C(R¹)₂ miteinander ein Ringsystem bilden, führt dies zu Spirostrukturen. Die Ausbildung derartiger Spirostrukturen durch Bildung von Ringsystemen zwischen zwei Gruppen R¹ an C(R¹)₂ ist eine weitere bevorzugte Ausführungsform der Erfindung. Dies gilt insbesondere, wenn R¹ für eine substituierte oder unsubstituierte Phenylgruppe steht und die beiden Phenylgruppen zusammen mit dem C-Atom der Brücke ein Ringsystem bilden.

Beispiele für bevorzugte Verbindungen gemäß den Formeln (1) bis (15) bzw. (3a) bis (15a) bzw. (3b) bis (15b) bzw. (3c) bis (15c) sind die im Folgenden abgebildeten Strukturen (1) bis (134) (*nicht erfindungsgmäß).

| | |
|---|---|
| 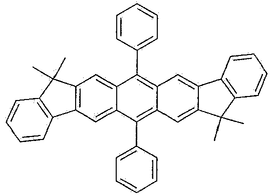 | 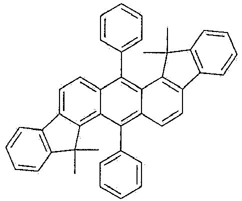 |
| (1) | (2) |
| 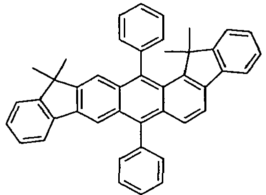 | 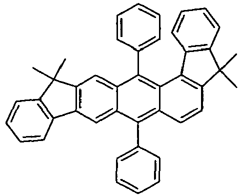 |
| (3) | (4) |
| 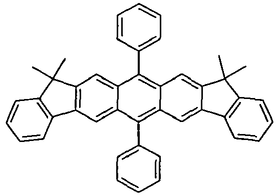 | 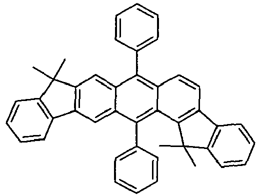 |
| (5) | (6) |
| 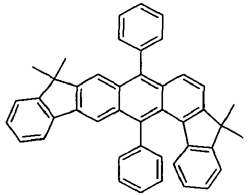 | 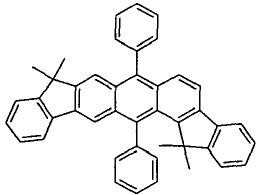 |
| (7) | (8) |
| 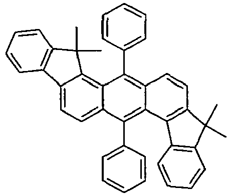 | 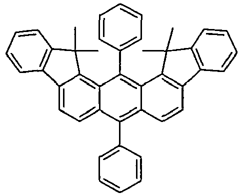 |
| (9) | (10) |
| 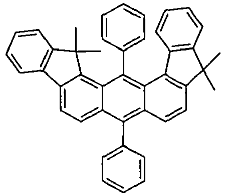 | 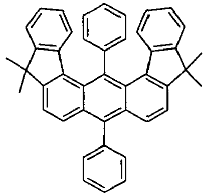 |
| (11) | (12) |
| 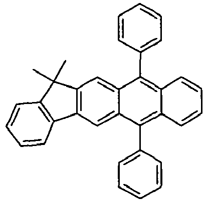 | 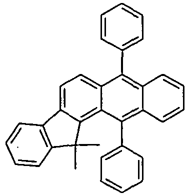 |
| (13) | (14) |
| 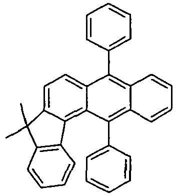 | 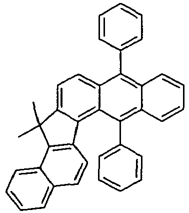 |
| (15) | (16) |
| 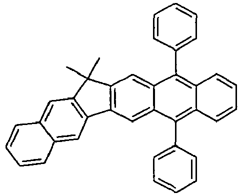 | 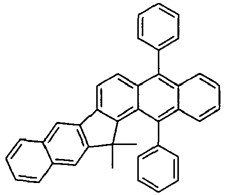 |
| (17) | (18) |
| 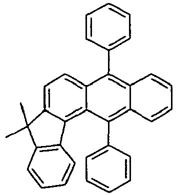 | 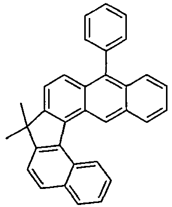 |
| (19) | (20) |
| 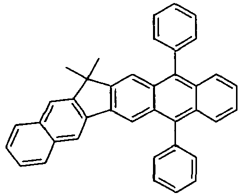 | 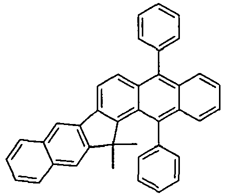 |
| (21) | (22) |
| 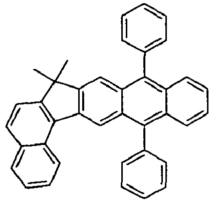 | 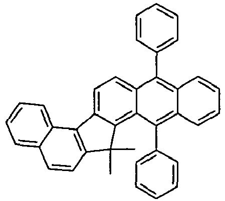 |
| (23) | (24) |
| 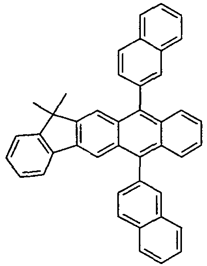 | 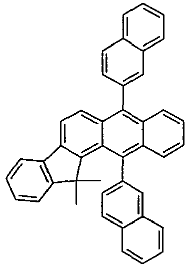 |
| (25) | (26) |
| 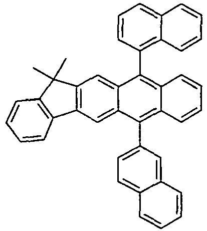 | 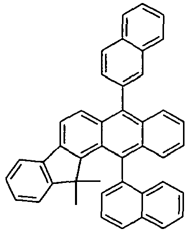 |
| (27) | (28) |
| 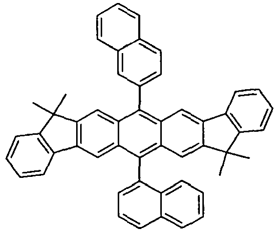 | 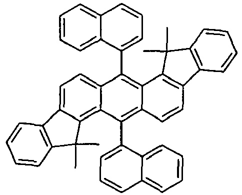 |
| (29) | (30) |
| 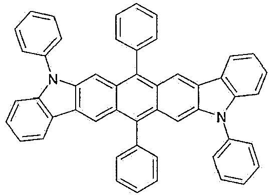 | 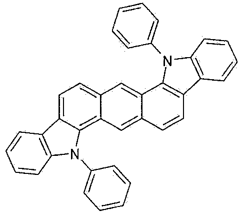 |
| (31)* | (32)* |
| 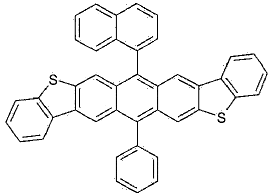 | 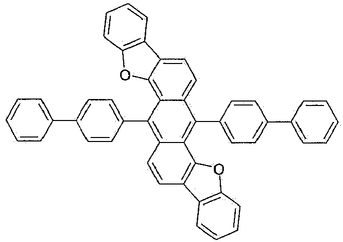 |
| (33)* | (34)* |
| 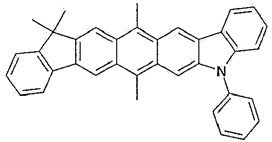 | 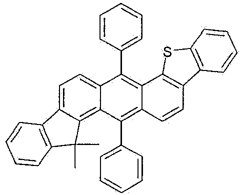 |
| (35)* | (36)* |
| 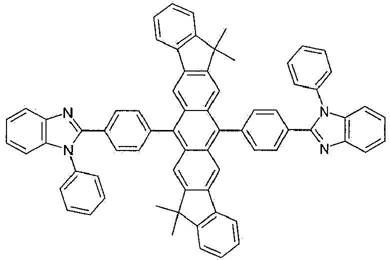 | 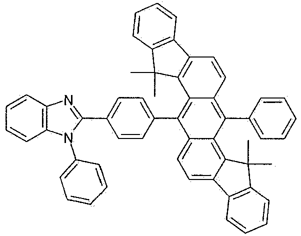 |
| (37)* | (38)* |
| 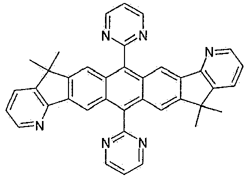 | 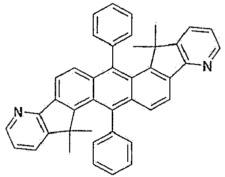 |
| (39) | (40) |
| 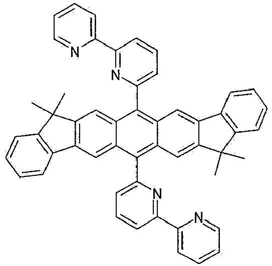 | 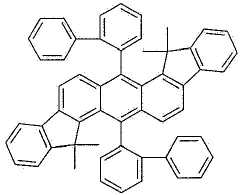 |
| (41) | (42) |
| 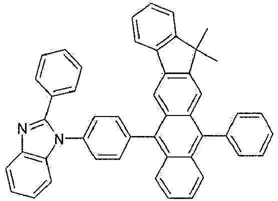 | 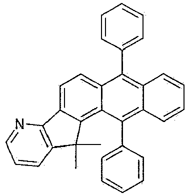 |
| (43)* | (44) |
| 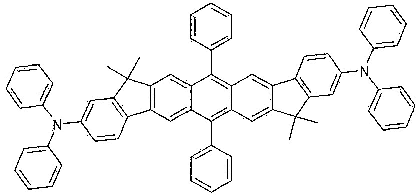 | 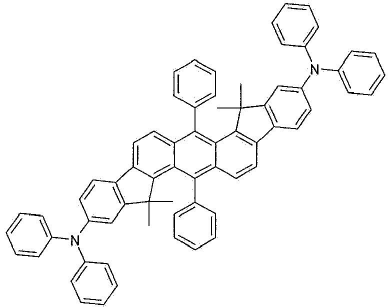 |
| (45)* | (46)* |
| 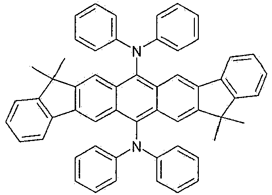 | 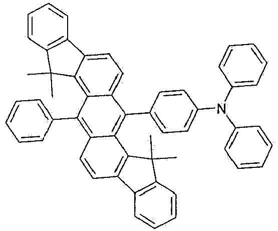 |
| (47)* | (48)* |
| 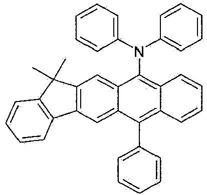 | 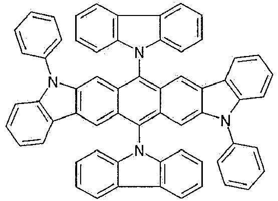 |
| (49)* | (50)* |
| 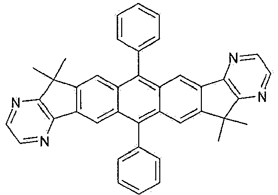 | 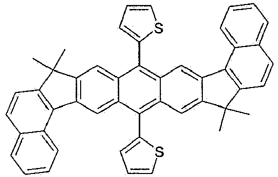 |
| (51) | (52) |
| 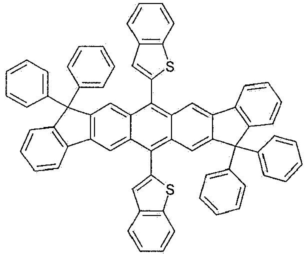 | 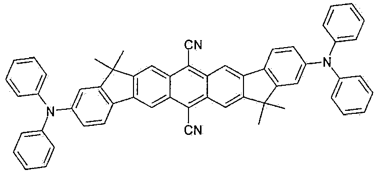 |
| (53) | (54)* |
| 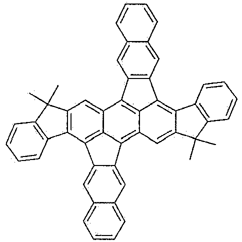 | 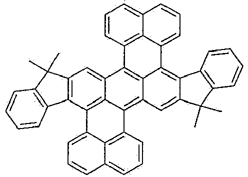 |
| (55)* | (56)* |
| 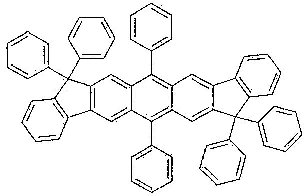 | 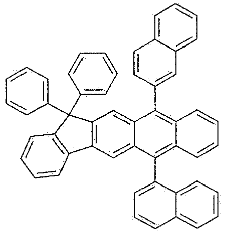 |
| (57) | (58) |
| 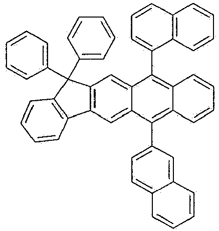 | 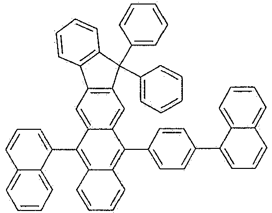 |
| (59) | (60)* |
| 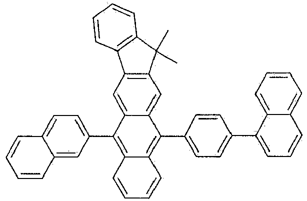 | 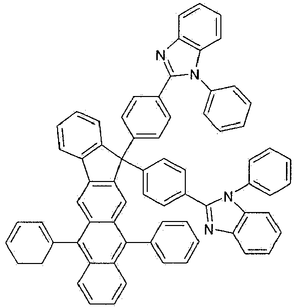 |
| (61)* | (62)* |
| 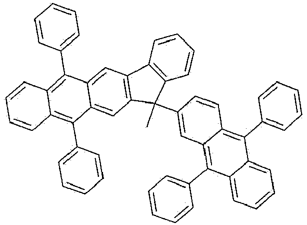 | 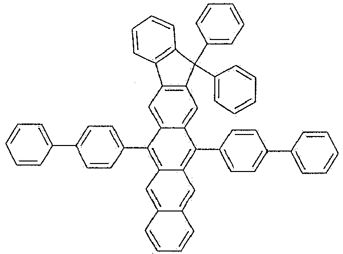 |
| (63)* | (64)* |
| 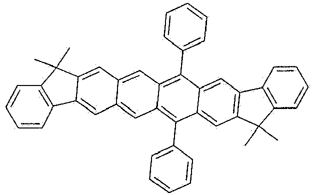 | 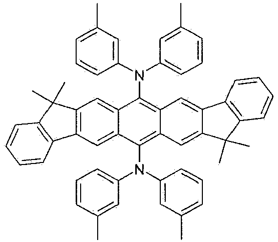 |
| (65)* | (66)* |
| 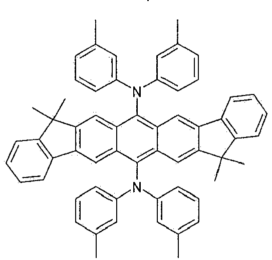 | 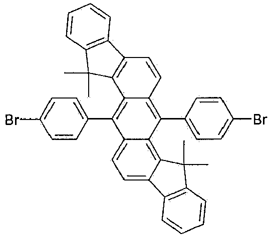 |
| (67)* | (68)* |
| 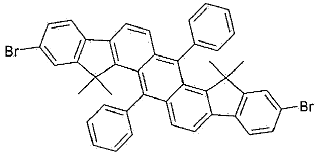 | 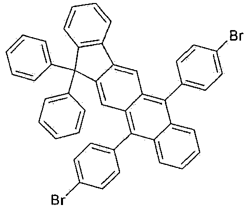 |
| (69)* | (70)* |
| 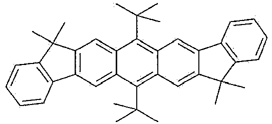 | 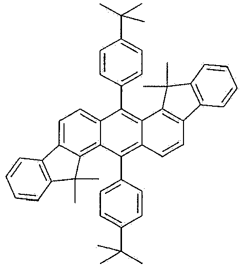 |
| (71)* | (72) |
| 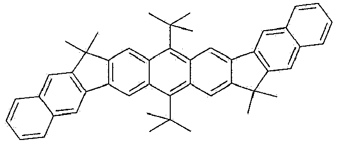 | 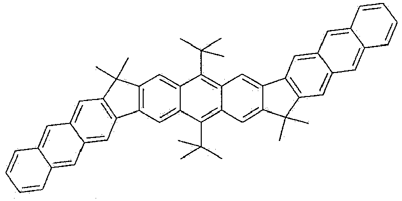 |
| (73)* | (74)* |
| 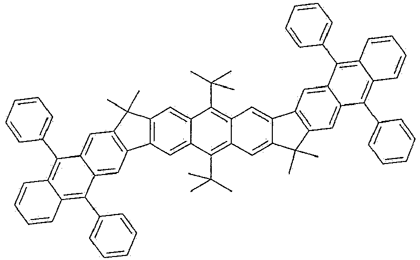 | 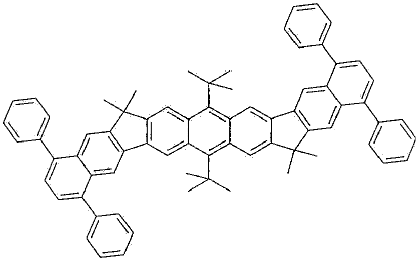 |
| (75)* | (76)* |
| 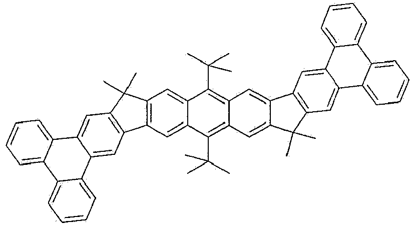 | 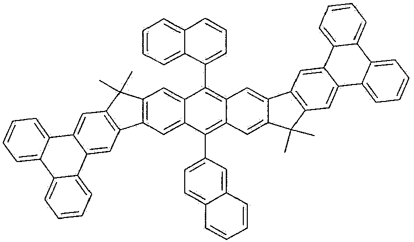 |
| (77)* | (78)* |
| 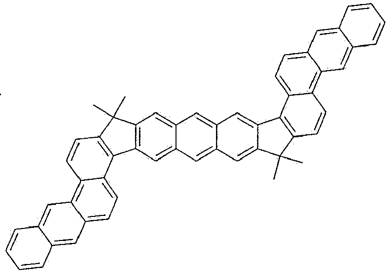 | 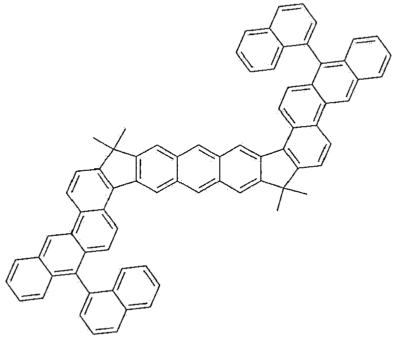 |
| (79)* | (80)* |
| 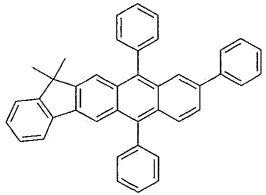 | 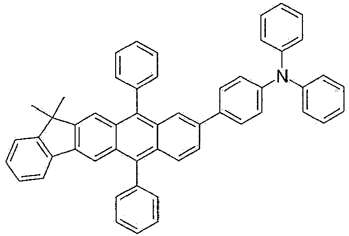 |
| (81)* | (82)* |
| 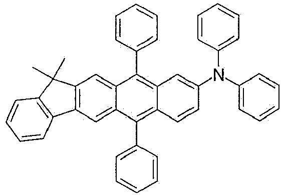 | 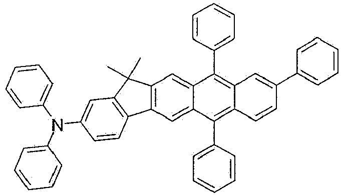 |
| (83)* | (84)* |
| 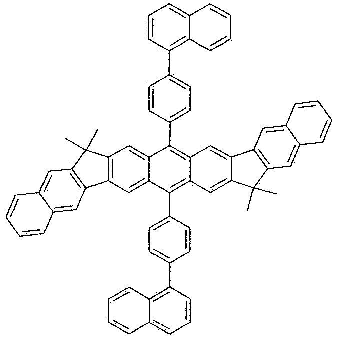 | 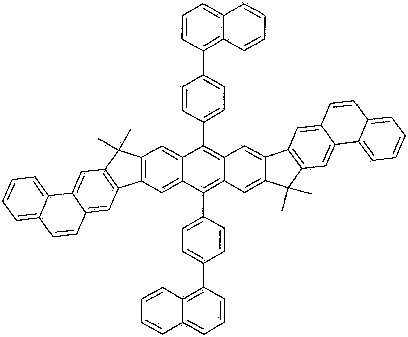 |
| (85)* | (86)* |
| 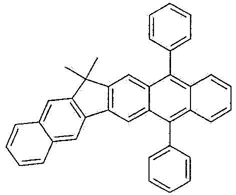 | 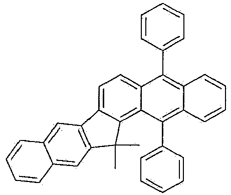 |
| (87) | (88) |
| 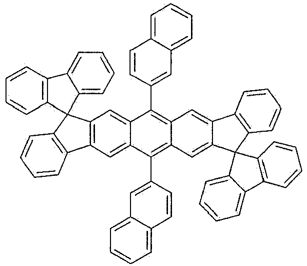 | 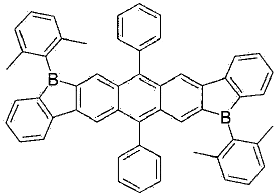 |
| (89) | (90)* |
| 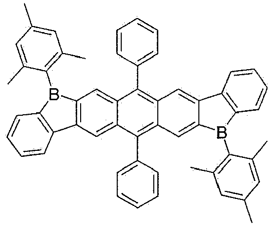 | 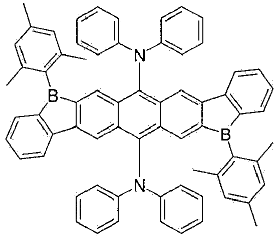 |
| (91)* | (92)* |
| 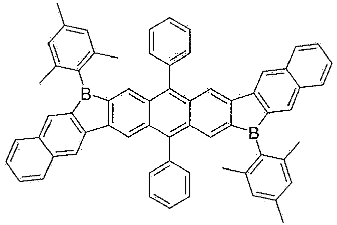 | 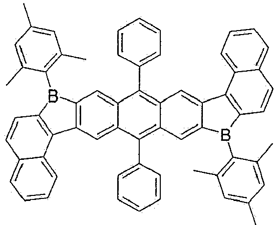 |
| (93)* | (94)* |
| 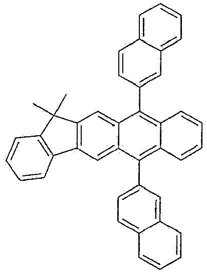 | 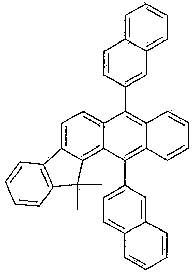 |
| (95) | (96) |
| 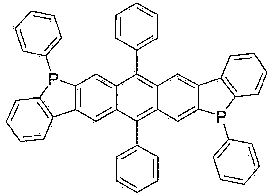 | 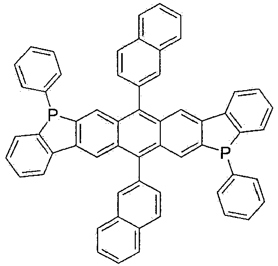 |
| (97)* | (98)* |
| 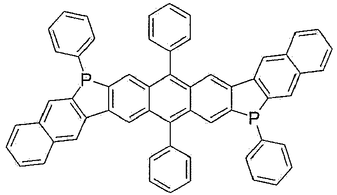 | 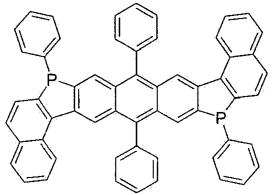 |
| (99)* | (100)* |
| 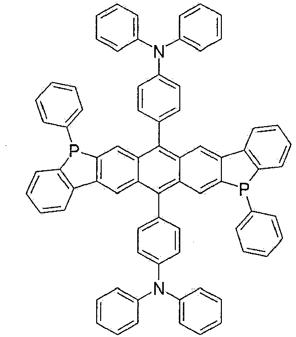 | 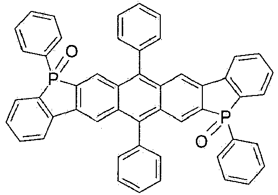 |
| (101)* | (102)* |
| 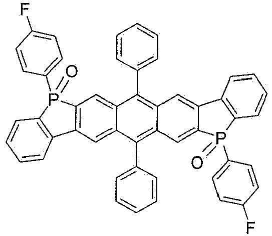 | 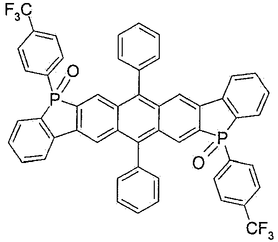 |
| (103)* | (104)* |
| 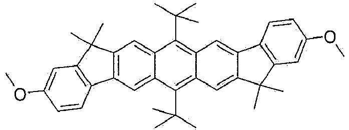 | 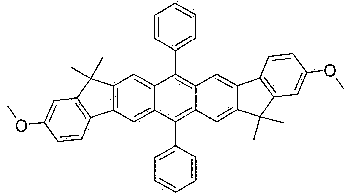 |
| (105)* | (106)* |
| 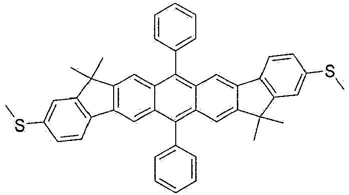 | 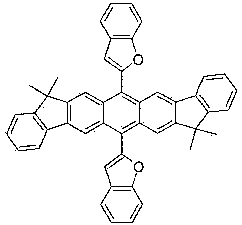 |
| (107)* | (108) |
| 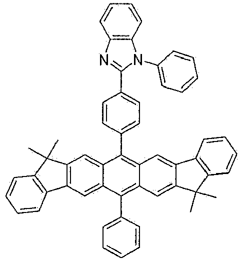 | 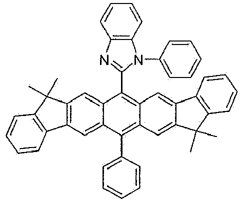 |
| (109)* | (110)* |
| 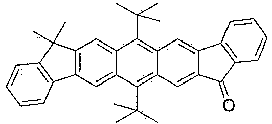 | 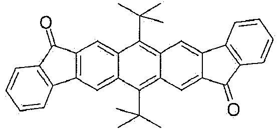 |
| (111)* | (112)* |
| 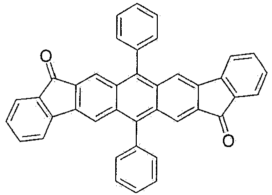 | 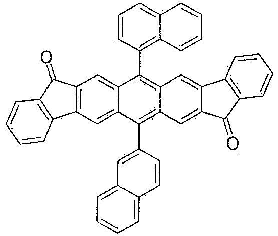 |
| (113)* | (114)* |
| 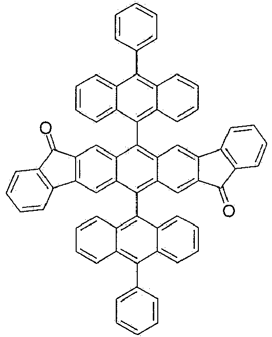 | 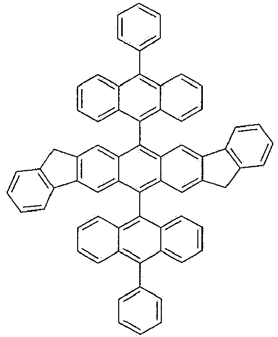 |
| (115)* | (116)* |
| 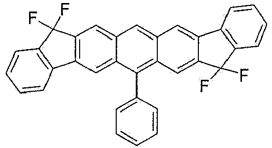 | 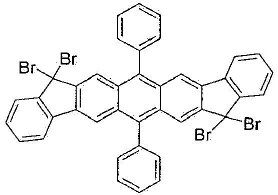 |
| (117)* | (118)* |
| 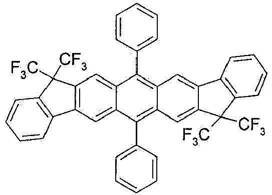 | 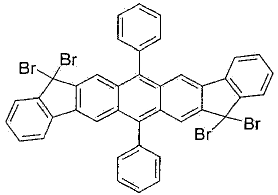 |
| (119) | (120)* |
| 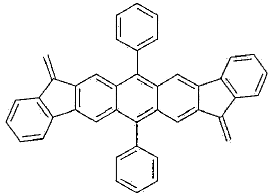 | 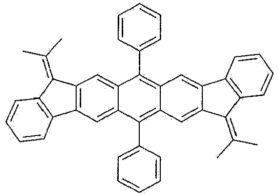 |
| (121)* | (122)* |
| 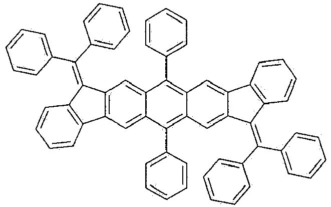 | 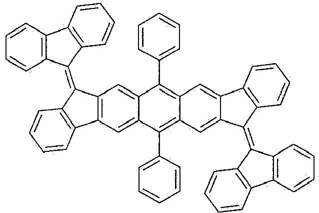 |
| (123)* | (124)* |
| 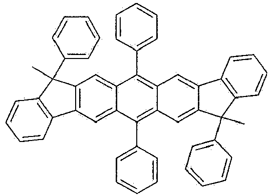 | 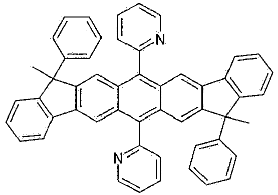 |
| (125) | (126) |
| 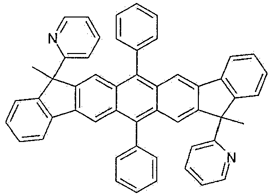 | 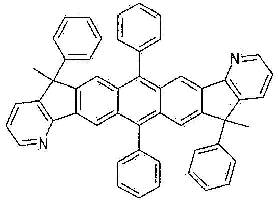 |
| (127)* | (128) |
| 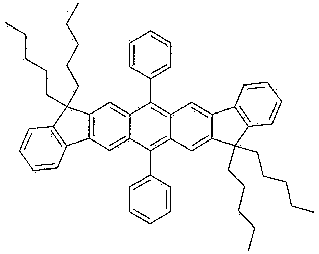 | 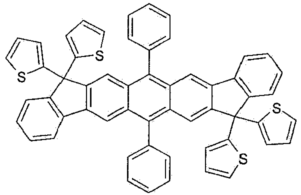 |
| (129)* | (130)* |
| 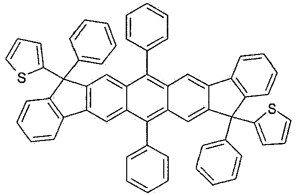 | 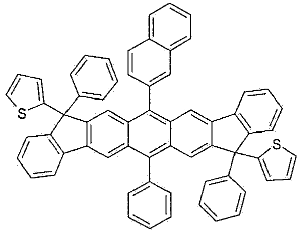 |
| (131)* | (132)* |
| 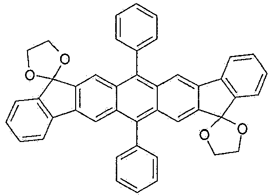 | 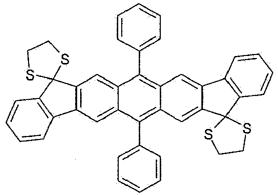 |
| (133)* | (134)* |

Die erfindungsgemäßen Verbindungen gemäß Formel (1) und (2) können nach dem Fachmann bekannten Syntheseschritten hergestellt werden. Die Synthese ist im folgenden Schema 1 beispielhaft abgebildet. Als Ausgangsverbindung eignet sich 2,6-Dibromanthrachinon, welches aus 2,6-Diaminoanthrachinon durch Diazotierung und Umsetzung mit CuBr₂ erhalten werden kann. Die Reste R können in Form einer Arylmetallverbindung, beispielsweise einer Aryllithiumverbindung oder einer Aryl-Grignard-Verbindung eingeführt werden, gefolgt von Reduktion der gebildeten Alkohole. Das Bromid kann durch Umsetzung mit einer Diborverbindung, beispielsweise Bis(pinacolato)dibor, unter Palladiumkatalyse in das entsprechende Boronsäurederivat umgewandelt werden, welches in einer Suzuki-Kupplung mit einem 2-Halogenalkylbenzoat umgesetzt werden kann. Die Reste an der Brücke X werden dann durch Umsetzung mit einer Aryllithiumverbindung oder einer Aryl-Grignard-Verbindung eingeführt, und die Cyclisierung zur Verbindung der Formel (1) bzw. (2) erfolgt unter sauren Bedingungen. Je nach den genauen Cyclisierungsbedingungen können bei der Cyclisierung auch Gemische entstehen, welche entweder getrennt werden können oder auch als Gemisch in der organischen Elektrolumineszenzvorrichtung eingesetzt werden können.

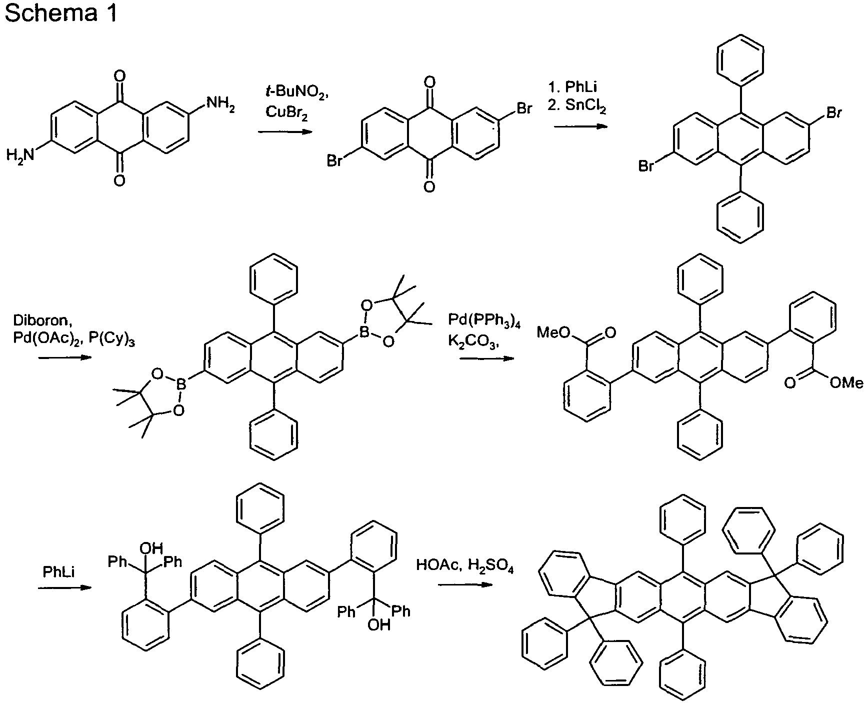

Analog hierzu lassen sich auch anders substituierte Verbindungen herstellen, bzw. es lassen sich andere Derivate der Formel (1) oder (2) herstellen, indem andere Dihalogenanthrachinone verwendet werden.

Ein weiterer Gegenstand der Erfindung ist ein Verfahren zur Herstellung der Verbindungen gemäß Formel (1) und (2) aus einem Dihalogenanthrachinon, umfassend die Reaktionsschritte:
a) Addition einer Arylmetallverbindung,
b) Reduktion zum Anthracen,
c) Kupplung mit einem funktionalisierten Aromaten, gegebenenfalls nach Umwandlung der Halogenfunktionalität in einer Boronsäurefunktionalität, und
d) Ringschluss, bevorzugt unter sauren Bedingungen.

Die oben beschriebenen erfindungsgemäßen Verbindungen, insbesondere Verbindungen, welche mit reaktiven Abgangsgruppen, wie Brom, lod, Boronsäure oder Boronsäureester, substituiert sind, können als Monomere zur Erzeugung entsprechender Dimere, Trimere, Tetramere, Pentamere, Oligomere, Polymere oder als Kern von Dendrimeren Verwendung finden. Die Oligomerisation bzw. Polymerisation erfolgt dabei bevorzugt über die Halogenfunktionalität bzw. die Boronsäurefunktionalität.

Weiterer Gegenstand der Erfindung sind daher Dimere, Trimere, Tetramere, Pentamere, Oligomere, Polymere oder Dendrimere enthaltend eine oder mehrere Verbindungen gemäß Formel (1) und/oder (2), wobei ein oder mehrere Reste R, R¹ oder R² Bindungen zwischen den Verbindungen gemäß Formel (1) bzw. (2) im Dimer, Trimer, Tetramer bzw. Pentamer bzw. Bindungen der Verbindung gemäß Formel (1) bzw. (2) zum Polymer, Oligomer oder Dendrimer darstellen. Unter einem Oligomer im Sinne dieser Erfindung wird eine Verbindung verstanden, welche mindestens sechs Einheiten gemäß Formel (1) und/oder (2) aufweist. Die Polymere, Oligomere oder Dendrimere können konjugiert, teilkonjugiert oder nichtkonjugiert sein. Die Trimere, Tetramere, Pentamere, Oligomere oder Polymere können linear oder verzweigt sein. In den linear verknüpften Strukturen können die Einheiten gemäß Formel (1) und/oder (2) sowohl direkt miteinander verknüpft sein oder sie können über eine bivalente Gruppe, beispielsweise über eine substituierte oder unsubstituierte Alkylengruppe, über ein Heteroatom oder über eine bivalente aromatische oder heteroaromatische Gruppe, miteinander verknüpft sein. In verzweigten Strukturen können beispielsweise drei oder mehrere Einheiten gemäß Formel (1) und/oder (2) über eine trivalente oder höhervalente Gruppe, beispielsweise über eine trivalente oder höhervalente aromatische oder heteroaromatische Gruppe, zu einem verzweigten Trimer, Tetramer, Pentamer, Oligomer oder Polymer verknüpft sein.

Für die Wiederholeinheiten gemäß Formel (1) und (2) in Dimeren, Trimeren, Tetrameren, Pentameren, Oligomeren und Polymeren gelten dieselben Bevorzugungen wie oben beschrieben.

Zur Herstellung der Oligomere oder Polymere werden die erfindungsgemäßen Monomere homopolymerisiert oder mit weiteren Monomeren copolymerisiert. Geeignete und bevorzugte Comonomere sind gewählt aus Fluorenen (z. B. gemäß EP 842208 oder WO 00/22026), Spirobifluorenen (z. B. gemäß EP 707020, EP 894107 oder WO 06/061181), Paraphenylenen (z. B. gemäß WO 92/18552), Carbazolen (z. B. gemäß WO 04/070772 oder WO 04/113468), Thiophenen (z. B. gemäß EP 1028136), Dihydrophenanthrenen (z. B. gemäß WO 05/014689), cis-und trans-Indenofluorenen (z. B. gemäß WO 04/041901 oder WO 04/113412), Ketonen (z. B. gemäß WO 05/040302), Phenanthrenen (z. B. gemäß WO 05/104264 oder WO 07/017066) oder auch mehreren dieser Einheiten. Die Polymere, Oligomere und Dendrimere enthalten üblicherweise noch weitere Einheiten, beispielsweise emittierende (fluoreszierende oder phosphoreszierende) Einheiten, wie z. B. Vinyltriarylamine (z. B. gemäß WO 07/068325) oder phosphoreszierende Metallkomplexe (z. B. gemäß WO 06/003000), und/oder Ladungstransporteinheiten, insbesondere solche basierend auf Triarylaminen.

Die erfindungsgemäßen Verbindungen gemäß Formel (1) und (2) eignen sich für den Einsatz in elektronischen Vorrichtungen, insbesondere in organischen Elektrolumineszenzvorrichtungen (OLEDs, PLEDs). Abhängig von der Substitution werden die Verbindungen in unterschiedlichen Funktionen und Schichten eingesetzt.

Ein weiterer Gegenstand der Erfindung ist daher die Verwendung von Verbindungen gemäß Formel (1) bzw. Formel (2) in elektronischen Vorrichtungen, insbesondere in organischen Elektrolumineszenzvorrichtungen.

Nochmals ein weiterer Gegenstand der Erfindung sind organische elektronische Vorrichtungen, enthaltend mindestens eine Verbindung gemäß Formel (1) und/oder (2), insbesondere organische Elektrolumineszenzvorrichtungen, enthaltend Anode, Kathode und mindestens eine emittierende Schicht, dadurch gekennzeichnet, dass mindestens eine organische Schicht, die eine emittierende Schicht oder eine andere Schicht sein kann, mindestens eine Verbindung gemäß Formel (1) und/oder (2) enthält.

Außer Kathode, Anode und der emittierenden Schicht kann die organische Elektrolumineszenzvorrichtung noch weitere Schichten enthalten. Diese sind beispielsweise gewählt aus jeweils einer oder mehreren Lochinjektionsschichten, Lochtransportschichten, Lochblockierschichten, Elektronentransportschichten, Elektroneninjektionsschichten, Elektronenblockierschichten, Charge-Generation Layers (IDMC 2003, Taiwan; Session 21 OLED (5), T. Matsumoto, T. Nakada, J. Endo, K. Mori, N. Kawamura, A. Yokoi, J. Kido, *Multiphoton Organic EL Device Having Charge Generation Layer*) und/oder organischen oder anorganischen p/n-Übergängen. Es sei aber darauf hingewiesen, dass nicht notwendigerweise jede dieser Schichten vorhanden sein muss und die Wahl der Schichten immer von den verwendeten Verbindungen abhängt und insbesondere auch von der Tatsache, ob es sich um eine fluoreszierende oder phosphoreszierende Elektrolumineszenzvorrichtung handelt.

In einer weiteren bevorzugten Ausführungsform der Erfindung enthält die organische Elektrolumineszenzvorrichtung mehrere emittierende Schichten, wobei mindestens eine organische Schicht mindestens eine Verbindung gemäß Formel (1) oder (2) enthält. Besonders bevorzugt weisen diese Emissionsschichten insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können und die blaues und gelbes, orange oder rotes Licht emittieren. Insbesondere bevorzugt sind Dreischichtsysteme, also Systeme mit drei emittierenden Schichten, wobei mindestens eine dieser Schichten mindestens eine Verbindung gemäß Formel (1) oder (2) enthält und wobei die drei Schichten blaue, grüne und orange oder rote Emission zeigen (für den prinzipiellen Aufbau siehe z. B. WO 05/011013). Ebenso eignen sich für weiße Emission Emitter, welche breitbandige Emissionsbanden aufweisen und dadurch weiße Emission zeigen.

In einer bevorzugten Ausführungsform der Erfindung werden die Verbindungen gemäß Formel (1) und (2) als Hostmaterial für fluoreszierende Dotanden eingesetzt, insbesondere für grün und rot fluoreszierende Dotanden. In diesem Fall ist bevorzugt die Gruppe X eine C(R¹)₂-Gruppe, die Gruppe Ar ist eine Arylgruppe und die Gruppen R sind H, eine Alkylgruppe oder eine Arylgruppe, wobei mindestens eine Gruppe R eine Alkyl- oder Arylgruppe darstellt; bevorzugt stellen beide Gruppen R Arylgruppen dar. Dieselben Bevorzugungen gelten für die Gruppen X, Ar und R in Strukturen gemäß Formel (3) bis (15), (3a) bis (15a), (3b) bis (15b) und (3c) bis (15c).

Unter einem Hostmaterial wird in einem System aus Host und Dotand diejenige Komponente verstanden, die in dem System im höheren Anteil vorliegt. Bei einem System aus einem Host und mehreren Dotanden wird als Host diejenige Komponente verstanden, deren Anteil der höchste in der Mischung ist.

Der Anteil des Hostmaterials gemäß Formel (1) bzw. (2) in der emittierenden Schicht beträgt zwischen 50.0 und 99.9 Vol.-%, bevorzugt zwischen 80.0 und 99.5 Vol.-%, besonders bevorzugt zwischen 90.0 und 99.0 Vol.-%. Entsprechend beträgt der Anteil des Dotanden zwischen 0.1 und 50.0 Vol.-%, bevorzugt zwischen 0.5 und 20.0 Vol.-%, besonders bevorzugt zwischen 1.0 und 10.0 Vol.-%.

Bevorzugte Dotanden in fluoreszierenden Vorrichtungen sind gewählt aus der Klasse der Monostyrylamine, der Distyrylamine, der Tristyrylamine, der Tetrastyrylamine und der Arylamine. Unter einem Monostyrylamin wird eine Verbindung verstanden, die eine Styrylgruppe und mindestens ein Amin enthält, welches bevorzugt aromatisch ist. Unter einem Distyrylamin wird eine Verbindung verstanden, die zwei Styrylgruppen und mindestens ein Amin enthält, welches bevorzugt aromatisch ist. Unter einem Tristyrylamin wird eine Verbindung verstanden, die drei Styrylgruppen und mindestens ein Amin enthält, welches bevorzugt aromatisch ist. Unter einem Tetrastyrylamin wird eine Verbindung verstanden, die vier Styrylgruppen und mindestens ein Amin enthält, welches bevorzugt aromatisch ist. Unter einem Arylamin bzw. einem aromatischen Amin im Sinne dieser Erfindung wird eine Verbindung verstanden, die drei aromatische oder heteroaromatische Ringsysteme direkt an den Stickstoff gebunden enthält, davon bevorzugt mindestens ein kondensiertes Ringsystem mit mindestens 14 aromatischen Ringatomen. Die Styrylgruppen sind besonders bevorzugt Stilbene, die auch an der Doppelbindung oder an den Aromaten weiter substituiert sein können. Beispiele für derartige Dotanden sind substituierte oder unsubstituierte Tristilbenamine oder weitere Dotanden, die beispielsweise in WO 06/000388, WO 06/058737, WO 06/000389, WO 07/065549 und WO 07/115610 beschrieben sind. Weiterhin sind Verbindungen gemäß WO 06/122630 als Dotanden bevorzugt. Beispiele für Arylamine sind Diarylaminoanthracene, wobei die Diarylaminogruppe in 2- oder 9-Position gebunden ist, Bis(diarylaminoanthracene), wobei die Diarylaminogruppen in 2,6- oder in 9,10-Position gebunden sind, Diarylaminopyrene, Bis(diarylamino)pyrene, Diarylaminochrysene oder Bis(diarylamino)chrysene. Bevorzugte Dotanden sind weiterhin Diarylaminderivate oder Bis(diarylamin)derivate von Monobenzoindenofluoren oder Dibenzoindenofluoren, beispielsweise gemäß WO 08/006449 oder WO 07/140847.

Geeignete Dotanden, für welche die Verbindung gemäß Formel (1) bzw. Formel (2) ein geeignetes Hostmaterial sind, sind weiterhin die in der folgenden Tabelle abgebildeten Strukturen sowie die in JP 06/001973, WO 04/047499, WO 06/098080, WO 07/065678, US 2005/0260442 und WO 04/092111 offenbarten Derivate dieser Strukturen.

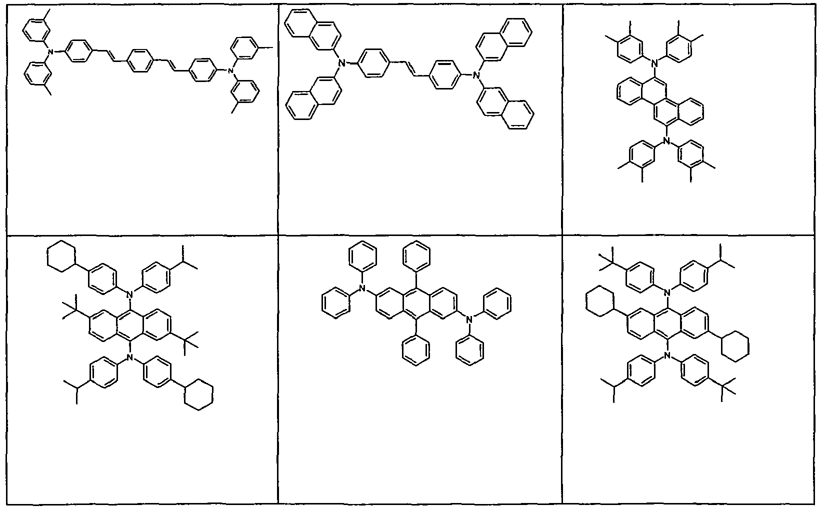

In einer weiteren bevorzugten Ausführungsform der Erfindung werden die Verbindungen gemäß Formel (1) und (2) als emittierende Materialien eingesetzt. Die Verbindungen sind insbesondere dann bevorzugt, wenn die Reste R für ein aromatisches oder heteroaromatisches Ringsystem stehen. Dies führt zu emittierenden Verbindungen, welche tiefblaue Emission mit einem sehr engen Emissionsspektrum zeigen. Die Verbindungen sind weiterhin dann als emittierende Verbindungen geeignet, wenn mindestens ein Substituent R mindestens eine Vinylaryl-Einheit, mindestens eine Vinylarylamin-Einheit und/oder mindestens eine Arylamino- bzw. Diarylamino-Einheit enthält. Bevorzugte Arylaminoeinheiten sind die Gruppen der vorne abgebildeten Formeln (16) und (17). Dieselben Bevorzugungen gelten für die Gruppen R in Strukturen gemäß Formel (3) bis (15) und (3a) bis (15a) bzw. (3b) bis (15b) bzw. (3c) bis (15c). Besonders bevorzugte Dotanden sind solche, in welchen entweder zwei Reste R für Gruppen der Formel (16) oder (17) stehen oder in welchen ein Rest R für eine Gruppe der Formel (16) oder (17) steht und der andere Reste R für H, eine Alkylgruppe oder eine Arylgruppe stehen.

Der Anteil der Verbindung gemäß Formel (1) bzw. (2) in der Mischung der emittierenden Schicht beträgt zwischen 0.1 und 50.0 Vol.-%, bevorzugt zwischen 0.5 und 20.0 Vol.-%, besonders bevorzugt zwischen 1.0 und 10.0 Vol.-%. Entsprechend beträgt der Anteil des Hostmaterials zwischen 50.0 und 99.9 Vol.-%, bevorzugt zwischen 80.0 und 99.5 Vol.-%, besonders bevorzugt zwischen 90.0 und 99.0 Vol.-%.

Als Hostmaterialien kommen hierfür Materialien verschiedener Stoffklassen in Frage. Bevorzugte Hostmaterialien sind ausgewählt aus den Klassen der Oligoarylene (z. B. 2,2',7,7'-Tetraphenylspirobifluoren gemäß EP 676461 oder Dinaphthylanthracen), insbesondere der Oligoarylene enthaltend kondensierte aromatische Gruppen, der Oligoarylenvinylene (z. B. DPVBi oder Spiro-DPVBi gemäß EP 676461), der polypodalen Metallkomplexe (z. B. gemäß WO 04/081017), der lochleitenden Verbindungen (z. B. gemäß WO 04/058911), der elektronenleitenden Verbindungen, insbesondere Ketone, Phosphinoxide, Sulfoxide, etc. (z. B. gemäß WO 05/084081 und WO 05/084082), der Atropisomere (z. B. gemäß WO 06/048268), der Boronsäurederivate (z. B. gemäß

WO 06/117052) oder der Benzanthracenderivate (z. B. gemäß WO 08/145239). Weiterhin kommen als Hostmaterialien auch die oben beschriebenen erfindungsgemäßen Verbindungen in Frage. Besonders bevorzugte Hostmaterialien sind außer den erfindungsgemäßen Verbindungen ausgewählt aus den Klassen der Oligoarylene, enthaltend Naphthalin, Anthracen, Benzanthracen und/oder Pyren oder Atropisomere dieser Verbindungen, der Oligoarylenvinylene, der Ketone, der Phosphinoxide und der Sulfoxide. Ganz besonders bevorzugte Hostmaterialien sind außer den erfindungsgemäßen Verbindungen ausgewählt aus den Klassen der Oligoarylene, enthaltend Anthracen, Benzanthracen und/oder Pyren oder Atropisomere dieser Verbindungen. Unter einem Oligoarylen im Sinne dieser Erfindung soll eine Verbindung verstanden werden, in der mindestens drei Aryl- bzw. Arylengruppen aneinander gebunden sind.

Geeignete Hostmaterialien sind weiterhin die in der folgenden Tabelle abgebildeten Materialien, sowie Derivate dieser Materialien, wie sie in WO 04/018587, WO 08/006449, US 5935721, US 2005/0181232, JP 2000/273056, EP 681019, US 2004/0247937 und US 2005/0211958 offenbart werden.

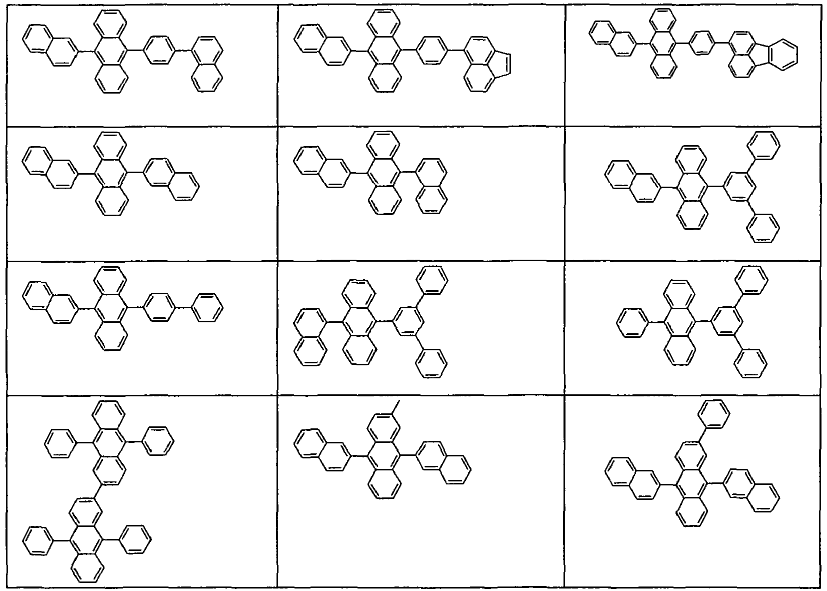

In nochmals einer weiteren Ausführungsform der Erfindung werden die Verbindungen gemäß Formel (1) und (2) als Lochtransportmaterial bzw. als Lochinjektionsmaterial eingesetzt. Die Verbindungen sind dann bevorzugt mit mindestens einer Gruppe N(Ar¹)₂ substituiert, bevorzugt mit mindestens zwei Gruppen N(Ar¹)₂ und/oder sie enthalten weitere Gruppen, die den Lochtransport verbessern. Die Gruppen N(Ar¹)₂ sind bevorzugt ausgewählt aus den oben beschriebenen Formeln (16) oder (17). Dies gilt insbesondere für die Reste R an den Strukturen gemäß Formel (3) bis (15) bzw. (3a) bis (15a) bzw. (3b) bis (15b) bzw. (3c) bis (15c). Weitere bevorzugte Gruppen, die den Lochtransport verbessern, sind beispielsweise die Gruppen N(R¹), S oder O, insbesondere N(R¹) als verbrückende Einheit X oder elektronenreiche Heteroaromaten, insbesondere Thiophen, Pyrrol oder Furan als Gruppe Ar. Die Verbindung wird bevorzugt in einer Lochtransport- bzw. in einer Lochinjektionsschicht eingesetzt. Eine Lochinjektionsschicht im Sinne dieser Erfindung ist eine Schicht, die direkt an die Anode angrenzt. Eine Lochtransportschicht im Sinne dieser Erfindung ist eine Schicht, die zwischen einer Lochinjektionsschicht und einer Emissionsschicht liegt. Wenn die Verbindungen gemäß Formel (1) bzw. (2) als Lochtransport- bzw. als Lochinjektionsmaterial verwendet werden, kann es bevorzugt sein, wenn sie mit Elektronenakzeptor-Verbindungen dotiert werden, beispielsweise mit F₄-TCNQ oder mit Verbindungen, wie in EP 1476881 oder EP 1596445 beschrieben.

In nochmals einer weiteren Ausführungsform der Erfindung werden die Verbindungen gemäß Formel (1) und (2) als Elektronentransportmaterial eingesetzt. Hier ist es bevorzugt, wenn eine oder beide verbrückenden Gruppen X, bevorzugt beide, für C=O, P(=O), SO oder SO₂ stehen und die Substituenten R für H, eine Alkylgruppe, eine Arylgruppe oder eine Heteroarylgruppe, welche einen elektronenarmen Heterocyclus darstellt. Weiterhin ist es hier bevorzugt, wenn die verbrückenden Gruppen X für C(R¹)₂ stehen und ein oder beide Substituenten R einen elektronenarmen Heterocyclus enthalten, wie beispielsweise Imidazol, Pyrazol, Thiazol, Benzimidazol, Benzothiazol, Triazol, Oxadiazol, Benzothiadiazol, Phenanthrolin, etc. Dies gilt insbesondere für die Gruppen X und R an den Strukturen gemäß Formel (3) bis (14) bzw. (3a) bis (14a) bzw. (3b) bis (14b) bzw. (3c) bis (14c). Weiterhin kann es bevorzugt sein, wenn die Verbindung mit Elektronendonorverbindungen dotiert ist.

Geeignete Ladungstransportmaterialien, wie sie in der Lochinjektions- bzw. Lochtransportschicht oder in der Elektronentransportschicht der erfindungsgemäßen organischen Elektrolumineszenzvorrichtung verwendet werden können, sind außer den erfindungsgemäßen Materialiene beispielsweise die in Y. Shirota et al., Chem. Rev. 2007, 107(4), 953-1010 offenbarten Verbindungen oder andere Materialien, wie sie gemäß dem Stand der Technik in diesen Schichten eingesetzt werden.

Geeignete Lochtransport- oder Lochinjektionsmaterialien, die in der erfindungsgemäßen Elektrolumineszenzvorrichtung enthaltend eine Verbindung der Formel (1) oder (2) verwendet werden können, sind beispielsweise die in der folgenden Tabelle aufgeführten Materialien.

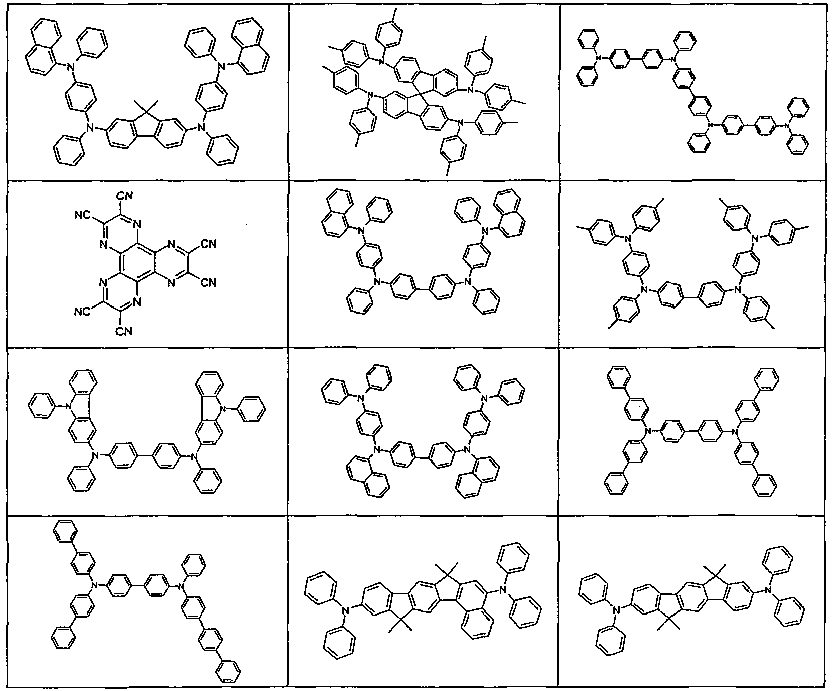

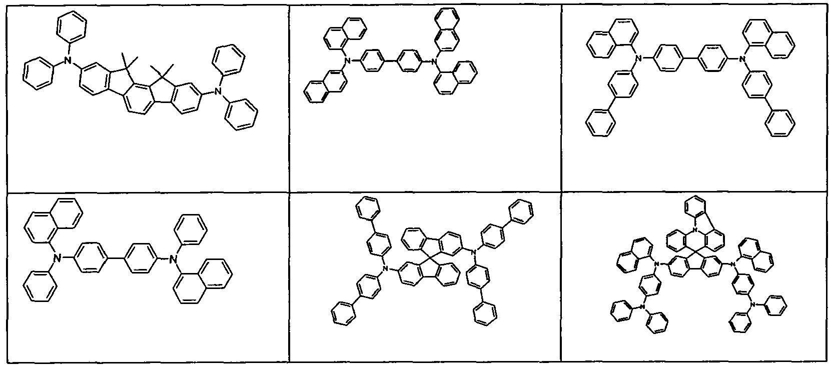

Weiterhin geeignete Lochtransport- und Lochinjektionsmaterialien sind Derivate der oben abgebildeten Verbindungen, wie sie in JP 2001/226331, EP 676461, EP 650955, WO 01/049806, US 4780536, WO 98/30071, EP 891121, EP 1661888, JP 2006/253445, EP 650955, WO 06/073054, US 5061569 und WO 06/122630 offenbart werden.

Geeignete Elektronentransport- oder Elektroneninjektionsmaterialien, die in der erfindungsgemäßen Elektrolumineszenzvorrichtung enthaltend eine Verbindung der Formel (1) oder (2) verwendet werden können, sind beispielsweise die in der folgenden Tabelle aufgeführten Materialien.

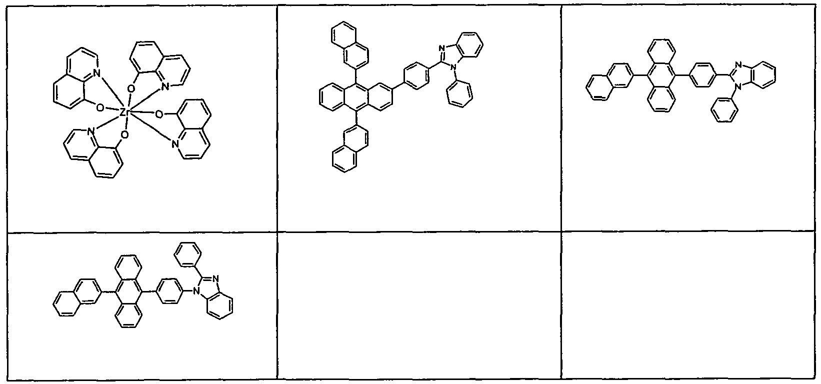

Weiterhin geeignete Elektronentransport- und Elektroneninjektionsmaterialien sind Derivate der oben abgebildeten Verbindungen, wie sie in JP 2000/053957, WO 03/060956, WO 04/028217 und WO 04/080975 offenbart werden.

Auch in Polymeren können Wiederholeinheiten gemäß Formel (1) und (2) entweder als Polymergrundgerüst (Backbone), als emittierende Einheit, als lochtransportierende Einheit und/oder als elektronentransportierende Einheit eingesetzt werden. Dabei entsprechen die bevorzugten Substitutionsmuster den oben beschriebenen.

Weiterhin bevorzugt ist eine organische Elektrolumineszenzvorrichtung, dadurch gekennzeichnet, dass eine oder mehrere Schichten mit einem Sublimationsverfahren beschichtet werden. Dabei werden die Materialien in Vakuum-Sublimationsanlagen bei einem Anfangsdruck von üblicherweise kleiner 10⁻⁵ mbar, bevorzugt kleiner 10⁻⁶ mbar aufgedampft. Es ist aber auch möglich, dass der Anfangsdruck noch geringer ist, beispielsweise kleiner 10⁻⁷ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck oder Offsetdruck, besonders bevorzugt aber LITI (Light Induced Thermal Imaging, Thermotransferdruck) oder Ink-Jet Druck (Tintenstrahldruck), hergestellt werden. Hierfür sind lösliche Verbindungen nötig. Hohe Löslichkeit lässt sich durch geeignete Substitution der Verbindungen erreichen.

Die erfindungsgemäßen Verbindungen weisen bei Verwendung in organischen Elektrolumineszenzvorrichtungen folgende überraschende Vorteile gegenüber dem Stand der Technik auf:
1. Die erfindungsgemäßen Verbindungen weisen eine hohe thermische Stabilität auf und lassen sich unzersetzt sublimieren.
2. Die erfindungsgemäßen Verbindungen, insbesondere solche, in denen R für ein aromatisches oder heteroaromatisches Ringsystem steht oder die mit Diarylaminosubstituenten substituiert sind, weisen sehr gute blaue bzw. grüne Farbkoordinaten auf und eignen sich daher sehr gut als blaue Emitter. Weiterhin weisen die Verbindungen einen geringen Stokes-Shift und ein sehr schmales Emissionsspektrum auf.
3. Die erfindungsgemäßen Verbindungen eignen sich sehr gut als Hostmaterial, insbesondere für rot und grün fluoreszierende Dotanden.
4. Die erfindungsgemäßen Verbindungen, insbesondere solche, welche mit Diarylaminogruppen substituitert sind und/oder welche S, O oder N(R¹) in der Brücke X enthalten und/oder welche elektronenreiche Heteroaromaten als Gruppe Ar enthalten, eignen sich sehr gut für die Verwendung als Lochinjektions- und Lochtransportmaterial und führen zu einer Verringerung der Betriebsspannung.
5. Die mit den erfindungsgemäßen Verbindungen hergestellten OLEDs weisen eine sehr hohe Lebensdauer auf.
6. Die mit den erfindungsgemäßen Verbindungen hergestellten OLEDs weisen eine sehr hohe Quanteneffizienz auf.

Im vorliegenden Anmeldetext wird auf die Verwendung der erfindungsgemäßen Verbindungen in Bezug auf OLEDs und PLEDs und die entsprechenden Displays abgezielt. Trotz dieser Beschränkung der Beschreibung ist es für den Fachmann ohne weiteres erfinderisches Zutun möglich, die erfindungsgemäßen Verbindungen auch in anderen elektronischen Vorrichtungen einzusetzen, z. B. in organischen Feld-Effekt-Transistoren (O-FETs), organischen Dünnfilmtransistoren (O-TFTs), organischen lichtemittierenden Transistoren (O-LETs), organischen integrierten Schaltungen (O-ICs), organischen Solarzellen (O-SCs), organischen Feld-Quench-Devices (O-FQDs), lichtemittierenden elektrochemischen Zellen (LECs), organischen Laserdioden (O-Laser) oder organischen Photorezeptoren.

Die Verwendung der erfindungsgemäßen Verbindungen in den entsprechenden Vorrichtungen ebenso wie diese Vorrichtungen selbst sind ebenfalls ein Gegenstand der vorliegenden Erfindung.

Die Erfindung wird durch die nachfolgenden Beispiele genauer beschrieben, ohne sie dadurch einschränken zu wollen.

### Beispiele

Die nachfolgenden Synthesen werden, sofern nicht anders angegeben, unter einer Schutzgasatmosphäre in getrockneten Lösungsmitteln durchgeführt. Die Edukte können von ALDRICH bezogen werden.

### Beispiel 1: 2,3,8,9-Dibenzo-1,1,7,7-tetraphenyl-5,11-diphenyl-1,7-dihydro-dicyclopenta[b,i]anthracen

### a) Synthese von 2,6-Dibromanthrachinon

### (Lee et al., Organic Letters 2005, 7(2), 323-326)

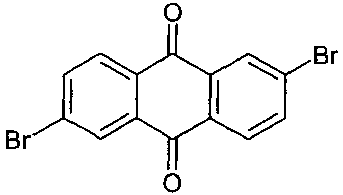

62.6 g (255 mmol) 2,6-Diaminoanthrachinon und 124 g (550 mmol) CuBr₂ werden in 1000 mL Acetonitril vorgelegt und 68 mL (515 mmol) *tert*-Butylnitrit bei 60 °C Innentemperatur zugetropft. Nach 30 min. wird das Reaktionsgemisch in ein Gemisch aus 150 mL konzentrierte HCl und 1 L Eiswasser gegossen, der entstandene Feststoff abgesaugt, mit Wasser, EtOH und Heptan gewaschen und getrocknet. Man erhält 89 g (243 mmol, 96 %) eines braunen Feststoffes, der im ¹H-NMR einheitlich ist und der ohne weitere Reinigung in die Folgereaktion eingesetzt wird.

### b) Synthese von 2,6-Dibrom-9,10-diphenylanthracen

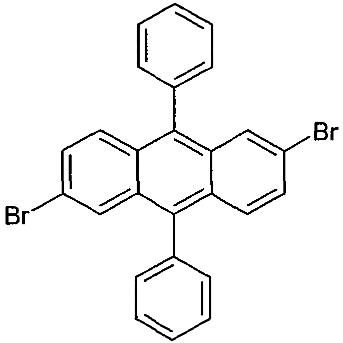

36.6 g (100 mmol) 2,6-Dibromanthrachinon werden in 600 mL THF gelöst, auf -75 °C abgekühlt und 100 mL einer 2 M Phenyllithiumlösung in THF zugetropft. Nach 2 h lässt man auf RT kommen, gibt 50 mL einer 4 M HCl zu, extrahiert zwischen Toluol und Wasser, trocknet die organische Phase über Na₂SO₄ und entfernt das Lösungsmittel im Vakuum. Der Rückstand wird in 350 mL DMF aufgenommen, 68 g (350 mmol) SnCl₂ zugegeben und für 2 h auf eine Innentemperatur von 140 °C gebracht. Danach gibt man bei ca. 40 °C 180 mL 2 M HCl zu, saugt den Niederschlag ab, wäscht mit Wasser, EtOH und Essigsäureethylester und trocknet bei 80 °C im Vakuum. Nach Umkristallisation aus Toluol erhält man 30.8 g (63 mmol, 63 %) eines ockerfarbenen Feststoffes, der nach DC- und ¹H-NMR einheitlich ist und in dieser Form in der Folgereaktion eingesetzt wird.

### c) Synthese von 9,10-Diphenylanthracen-2,6-diboronsäurepinacol-ester

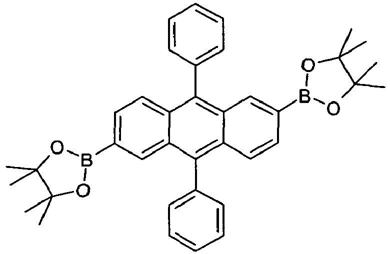

9.5 g (19.5 mmol) 2,6-Dibrom-9,10-diphenylanthracen werden in 240 mL trockenem Dioxan gelöst, 16.3 g (64 mmol) Bis(pinacolato)dibor, 1 g (1.2 mmol) [1,1'-Bis(diphenylphosphino)ferrocen]palladium(II)chlorid (Komplex mit Dichlormethan (1:1), Pd-Gehalt: 13 %) und 23 g (234 mmol) Kaliumacetat zugegeben, das Gemisch für 2 h zum Sieden erhitzt, in Eiswasser gegossen, der Niederschlag abgesaugt, mit EtOH gewaschen und im Vakuum getrocknet. Man erhält 10 g (17.1 mmol, 88 %) eines fahlgelben, DC- und ¹H-NMR einheitlichen Pulvers.

### d) Synthese von 9,10-Diphenyl-2,6-(2-carboxymethylphenyl)-anthracen

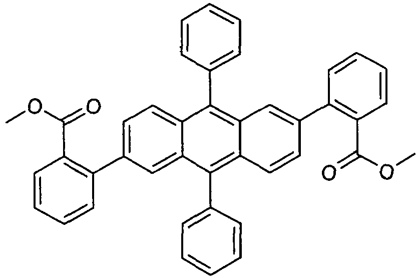

9.4 g (16 mmol) 9,10-Diphenylanthracen-2,6-diboronsäurepinacolester werden 4 h in einer Mischung aus 115 mL EtOH, 115 mL Toluol, 60 mL einer 2 M Na₂CO₃-Lösung, 4.5 mL (32.3 mmol) Methyl-2-brombenzoat und 750 mg (0.7 mmol) Pd(PPh₃)₄ zum Sieden erhitzt. Im Anschluss wird die Reaktionsmischung in eine Mischung aus Eiswasser/MeOH/HCl 1:1:1 gegossen, der farblose Niederschlag abgesaugt, mit Wasser, EtOH und Heptan gewaschen und getrocknet. Der Feststoff wird in siedendem Toluol gelöst, über eine Kieselgelschicht filtriert, das Filtrat mit Heptan versetzt und das ausgefallene Produkt abgesaugt. Man erhält 8.9 g (14.8 mmol, 92 %) des Diesters als farbloses Pulver.

### e) Synthese von (2-{6-[2-(Hydroxy-diphenyl-methyl)-phenyl]-9,10-diphenyl-anthracen-2-yl}-phenyl)-diphenyl-methanol

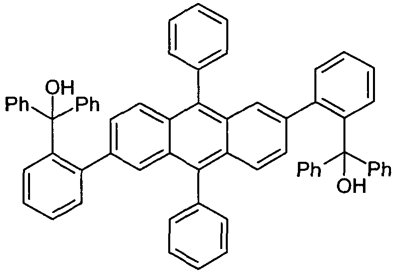

8.9 g (14.9 mmol) des Diesters werden bei RT mit 180 ml einer 0.5 M Phenylmagnesiumbromid-Lösung (90 mmol) in THF versetzt und für 3 h zum Sieden erhitzt. Nach dieser Zeit werden 50 mL 50%ige Essigsäure zugetropft, das Lösungsmittel im Vakuum entfernt, der verbliebene Feststoff in MeOH aufgenommen, abgesaugt und mit MeOH gewaschen und getrocknet. Es verbleiben 11.3 g (13.3 mmol, 90 %) eines farblosen Feststoffes, der nach DC und ¹H-NMR einen Reinheitsgrad von >98% aufwies.

### f) Synthese von Dibenzo-1,1,7,7-tetraphenyl-5,11-diphenyl-1,7-dihydro-dicyclopenta-[b,i]anthracen

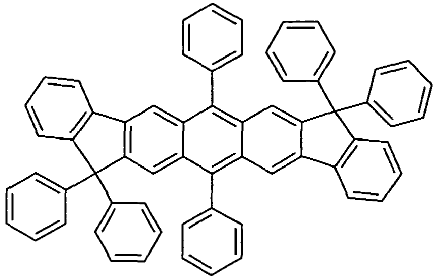

10.3 g (12.2 mmol) des Diols werden für 2 h in einer Mischung aus 70 mL Eisessig und 1 mL konz. HCl zum Sieden erhitzt. Nach erfolgtem Umsatz wird der ausgefallene Feststoff abgesaugt, mit Wasser, EtOH und Heptan gewaschen und getrocknet. Nach vierfacher Umkristallisation aus Chlorbenzol und zweifacher Sublimation im Vakuum (T = 340 °C, p = 1 x 10⁻⁵ mbar) verbleiben 6 g (7.4 mmol, 61 %) in Form eines blassgelben Glases mit einer durch RP-HPLC bestimmten Reinheit von >99.9%.

### Beispiel 2: 2,3-Benzo-1,1-diphenyl-5,11-diphenyl-1-dihydrocyclo-penta[b]anthracen

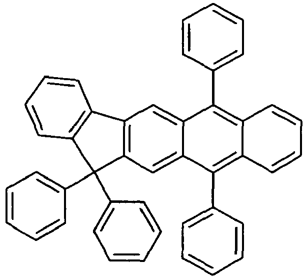

Die Synthese dieser Verbindung erfolgt analog zu Beispiel 1, wobei 2-Aminoanthrachinon als Ausgangsverbindung eingesetzt wird.

### Beispiel 3: Herstellung der OLEDs

Die Herstellung von OLEDs erfolgt nach einem Verfahren, das in WO 04/058911 allgemein beschrieben ist und das im Einzelfall auf die jeweiligen Gegebenheiten (z. B. Schichtdickenvariation, um optimale Effizienz bzw. Farbe zu erreichen) angepasst wird.

In den folgenden Beispielen 4 bis 7 werden die Ergebnisse verschiedener OLEDs vorgestellt. Glasplatten, die mit strukturiertem ITO (Indium Zinn Oxid) beschichtet sind, bilden die Substrate der OLEDs. Die OLEDs bestehen aus folgender Schichtenfolge: Substrat / Lochinjektionsschicht (HTM1) 60 nm / Lochtransportschicht (HTM2) 20 nm / Emissionschicht (EML) 30 nm / Elektronentransportschicht (ETM) 20 nm und abschließend eine Kathode. Die Materialien werden in einer Vakuumkammer thermisch aufgedampft. Dabei besteht die Emissionsschicht immer aus einem Matrixmaterial (Host) und einem Dotierstoff (Dotand), der durch Coverdampfung dem Host beigemischt wird. Die Kathode wird durch eine 1 nm dünne LiF-Schicht und einer darauf abgeschiedenen 100 nm AI-Schicht gebildet. Die Tabelle 1 zeigt die chemischen Strukturen der zum Aufbau der OLEDs verwendeten Materialien.

Diese OLEDs werden standardmäßig charakterisiert; hierfür werden die Elektrolumineszenzspektren, die Effizienz (gemessen in cd/A), die Leistungseffizienz (gemessen in lm/W) in Abhängigkeit der Helligkeit, berechnet aus Strom-Spannungs-Helligkeit-Kennlinien (IUL-Kennlinien), und die Lebensdauer bestimmt. Als Lebensdauer wird die Zeit definiert, nach der die Anfangshelligkeit von 25000 cd/m² auf die Hälfte gesunken ist. Aus den Elektrolumineszenzspektren wird die Halbwertsbreite (FWHM= full width half maximum) bestimmt.

In Tabelle 1 sind die Ergebnisse einiger OLEDs (Beispiele 4 bis 7) zusammengefasst. Als erfindungsgemäße Hostmaterialien bzw. Emittermaterialien werden die Verbindungen der Beispiele 1 und 2 verwendet. Als Vergleichsbeispiele werden der Host H1 bzw. das Emittermaterial D1 gemäß dem Stand der Technik verwendet.

Wie man aus den Ergebnissen in Tabelle 2 deutlich erkennen kann, weisen organische Elektrolumineszenzvorrichtungen enthaltend die erfindungsgemäßen Verbindungen eine höhere Lebensdauer im Fall des Matrixbeispiels auf. Wie man aus der Tabelle 3 erkennen kann, weisen organische Elektrolumineszenzvorrichtungen enthaltend die erfindungsgemäßen Verbindungen als Dotanden eine wesentlich verbesserte Halbwertsbreite im Vergleich zum Stand der Technik auf.

**Tabelle 1**

| | | |
|---|---|---|
| 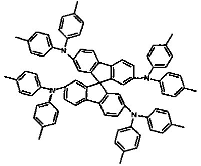 | 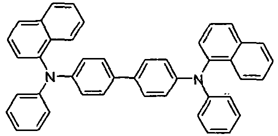 | 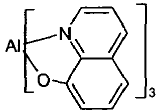 |
| HTM1 | HTM 2 | ETM1 |
| 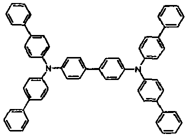 | 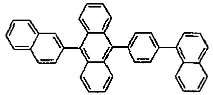 | 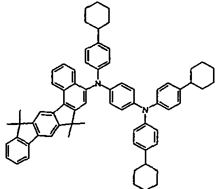 |
| HTM 3 | H1 | D1 |
| 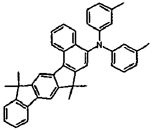 | 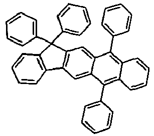 | 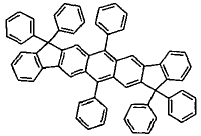 |
| D2 | Bsp. 2 | Bsp. 1 |

**Tabelle 2**

| **Bsp.** | **EML** | **ETM** | **Farbe** | **Max. Eff. (cd/A)** | **Spannung (V) bei 1000cd/m²** | **CIE** | **Lebensdauer bei 25000 cd/m² (h)** |
|---|---|---|---|---|---|---|---|
| 4 Vergleich | H1+5% D1 | ETM1 | grün | 18.3 | 5.3 | x=0.29/ y=0.60 | 300 |
| 5 | Bsp. 2 + 5% D1 | ETM1 | grün | 22.5 | 4.9 | x=0.29/ y=0.62 | 410 |

**Tabelle 3**

| **Bsp.** | **EML** | **ETM** | **Farbe** | **Max. Eff. (cd/A)** | **Spannung (V) bei 1000cd/m²** | **CIE** | **FWHM** |
|---|---|---|---|---|---|---|---|
| 6 Vergleich | H1+5% D2 | ETM1 | blau | 3.9 | 5.7 | x=0.14/ y=0.12 | 49 |
| 7 | H1 + 1% Bsp. 1 | ETM1 | blau | 3.1 | 5.7 | x=0.14/ y=0.09 | 24 |

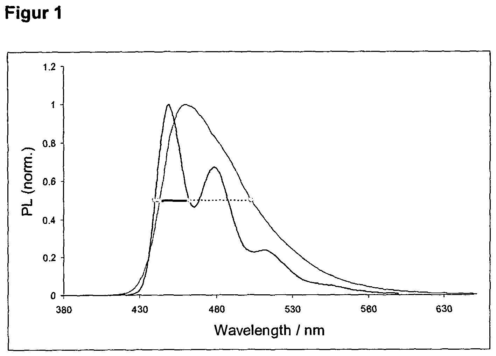

Die deutlich schmalere Halbwertsbreite des Emissionspeaks wird in Fig. 1 verdeutlicht. Darin werden die Photolumineszenzspektren und die Halbwertsbreite der oben abgebildeten Verbindung D2 (gepunktete Linie; Verbindung gemäß WO 08/006449) mit der erfindungsgemäßen Verbindung aus Bsp. 1 (durchgezogene Linie) in Toluol abgebildet.

## Patentansprüche

1. Verbindungen gemäß der Formel (1) und (2), 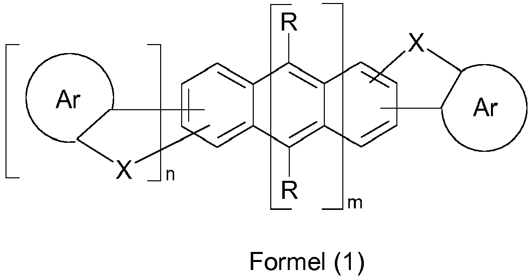 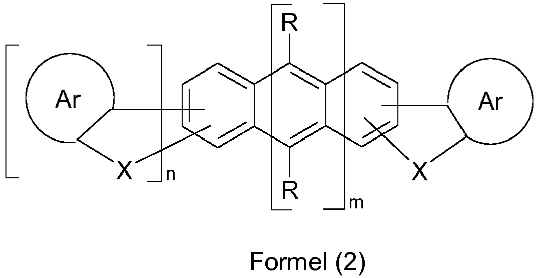 dabei können in der Anthraceneinheit auch ein oder mehrere unsubstituierte Kohlenstoffatome auch durch Stickstoff ersetzt sein; weiterhin gilt für die verwendeten Symbole und Indizes:
X ist bei jedem Auftreten gleich oder verschieden eine bivalente Brücke, ausgewählt aus C(R¹)₂,
Ar ist bei jedem Auftreten gleich oder verschieden eine Arylgruppe mit 6 bis 10 C-Atomen oder eine Heteroarylgruppe mit 4 bis 9 C-Atomen;
R¹ ist bei jedem Auftreten gleich oder verschieden methyl, ethyl, iso-Propyl, tert-Butyl, wobei ein H-Atom oder mehrere H-Atome durch F ersetzt sein können, oder eine Arylgruppe mit 6 bis 14 C-Atomen, die jeweils durch einen oder mehrere Reste R² substituiert sein kann; dabei können zwei oder mehrere benachbarte Substituenten R¹ auch miteinander ein mono- oder polycyclisches, aliphatisches oder aromatisches Ringsystem bilden;
R ist bei jedem Auftreten gleich oder verschieden ein aromatisches oder heteroaromatisches Ringsystem mit 5 bis 14 aromatischen Ringatomen, das jeweils durch einen oder mehrere Reste R² substituiert sein kann;
R² ist bei jedem Auftreten gleich oder verschieden H, D, F, Cl, Methyl, Ethyl, n-Propyl, i-Propyl, n-Butyl, i-Butyl, s-Butyl, t-Butyl, wobei ein oder mehrere H-Atome durch F, Cl, oder CN ersetzt sein können;
m ist 1;
n ist 0 oder 1;
**dadurch gekennzeichnet, dass** die Gruppen Ar unsubstituiert sind.

2. Verbindungen nach Anspruch 1 gemäß den Formeln (3) bis (15), 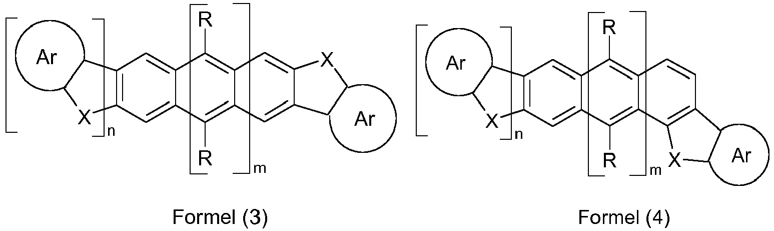 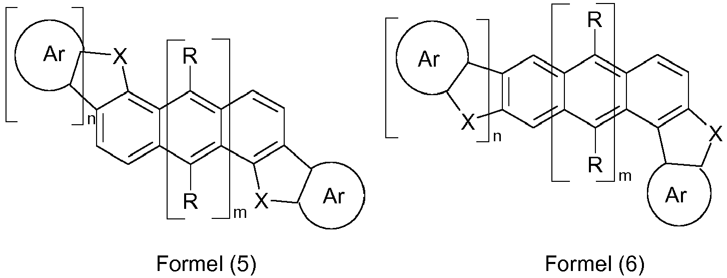 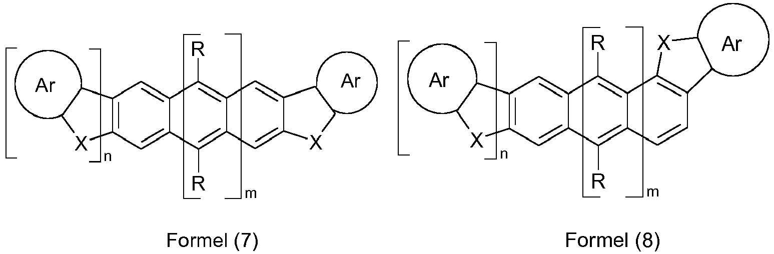 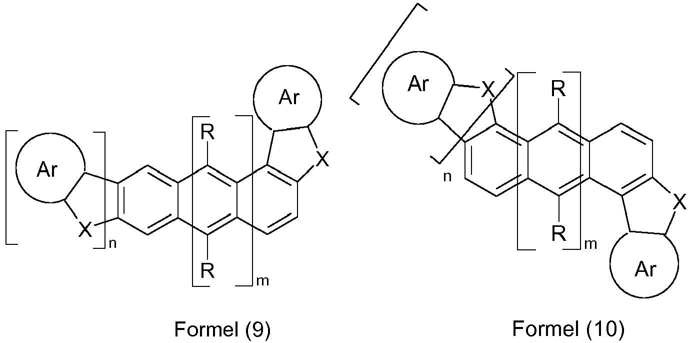 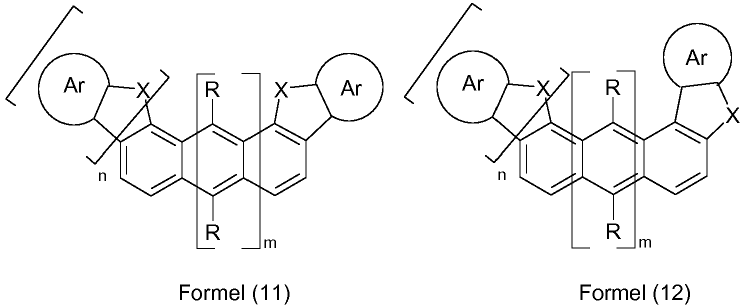 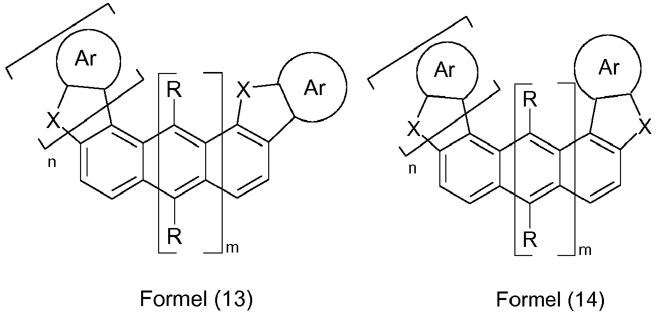 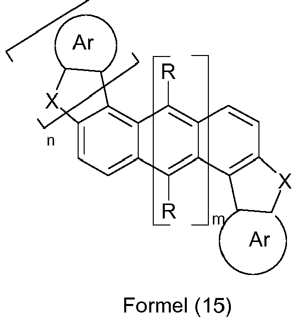 wobei in der Anthraceneinheit auch ein oder mehrere unsubstituierte Kohlenstoffatome durch Stickstoff ersetzt sein können und wobei die Symbole und Indizes die in Anspruch 1 aufgeführten Bedeutungen haben.

3. Verbindungen nach Anspruch 1 oder 2, **dadurch gekennzeichnet, dass** das Symbol Ar gleich oder verschieden bei jedem Auftreten für Benzol, Naphthalin, Thiophen, Pyrrol, Furan, Pyridin, Pyrimidin, Pyrazin, Pyridazin, Chinolin, Chinoxalin, Benzothiophen, Indol oder Benzofuran steht.

4. Verbindungen nach einem oder mehreren der Ansprüche 1 bis 3 gemäß den Formeln (3a) bis (15a), (3b) bis (15b) und (3c) bis (15c), 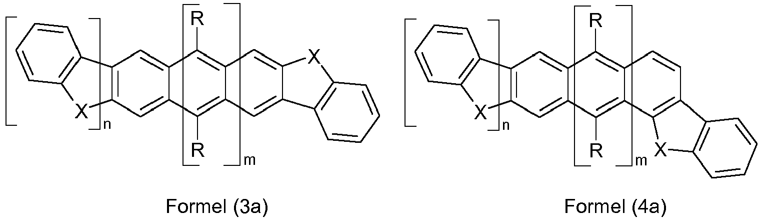 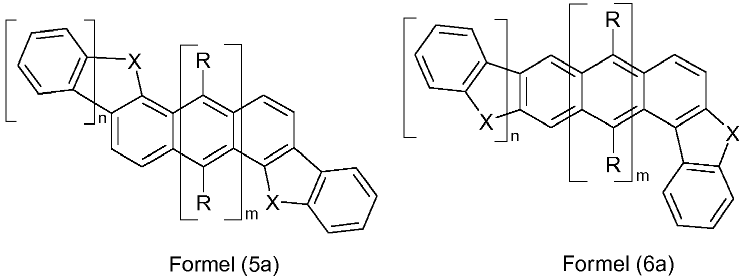 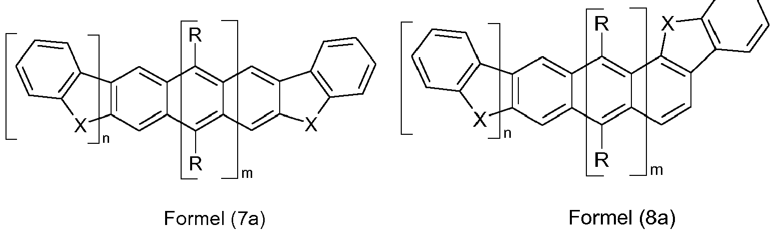 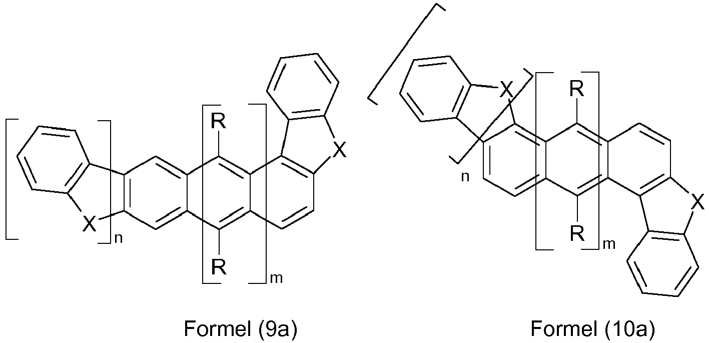 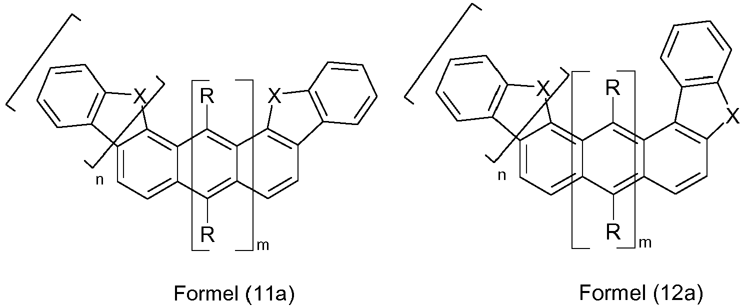 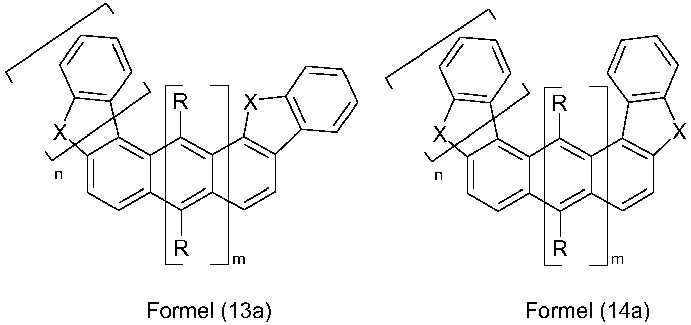 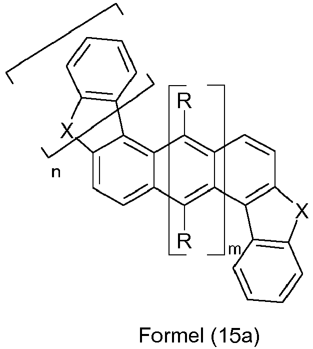 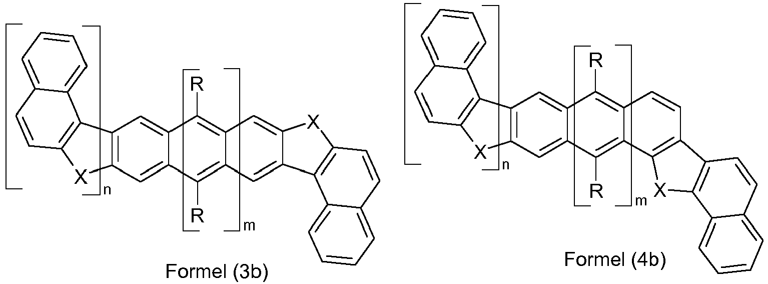 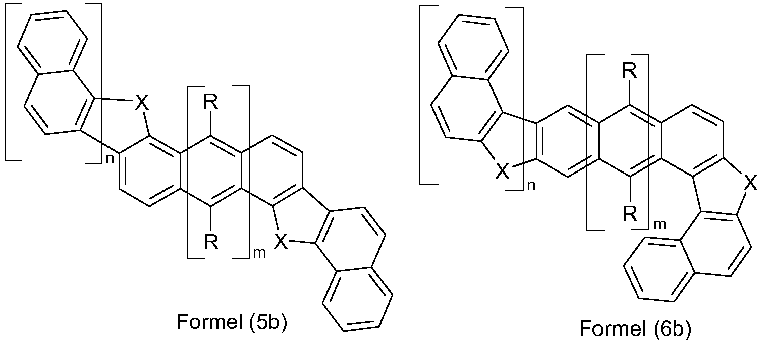 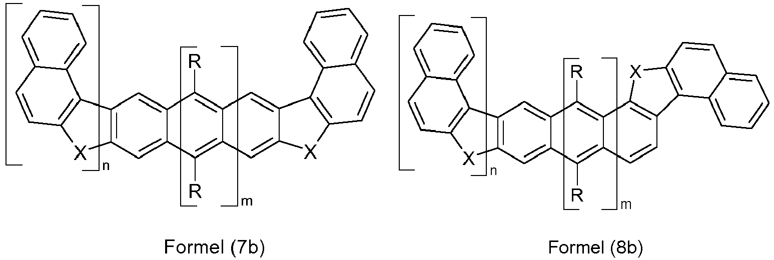 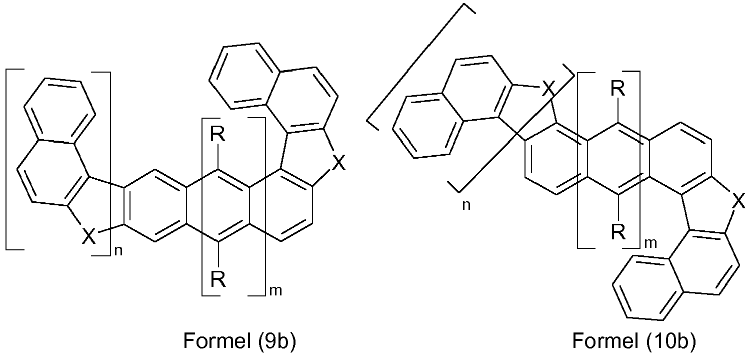 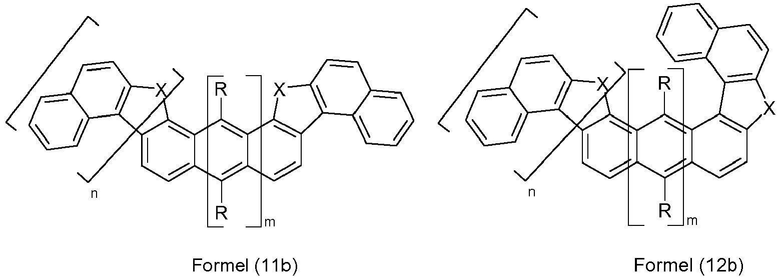 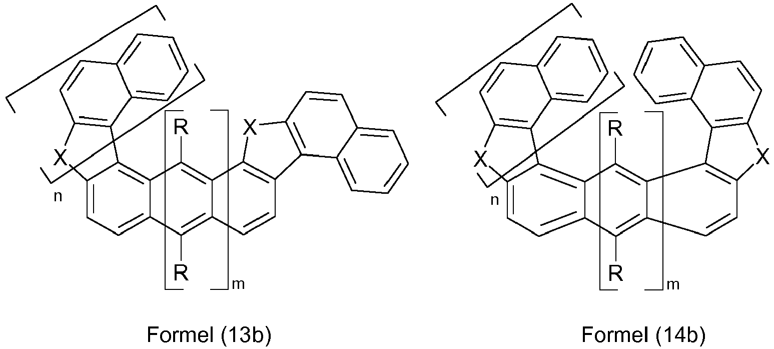 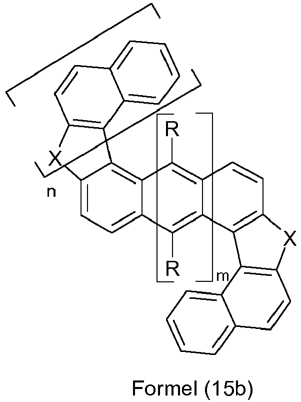 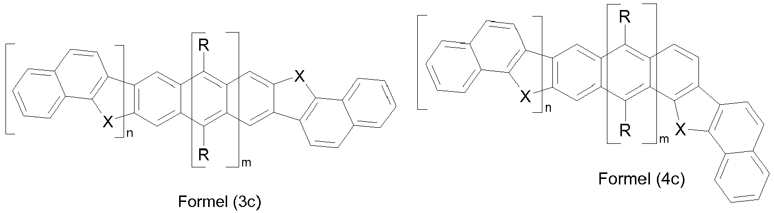 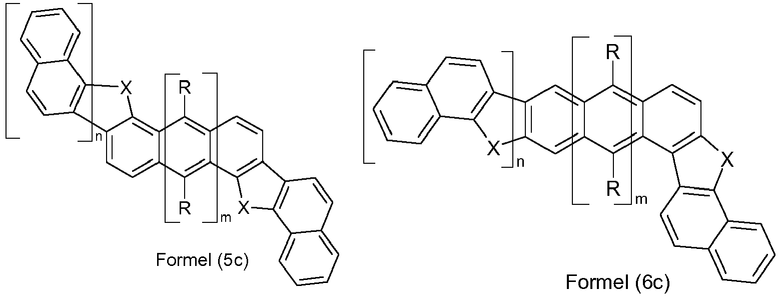 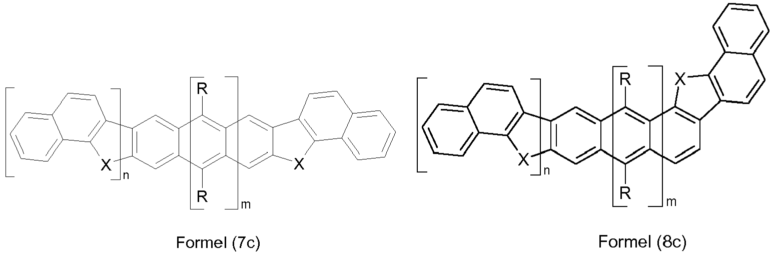 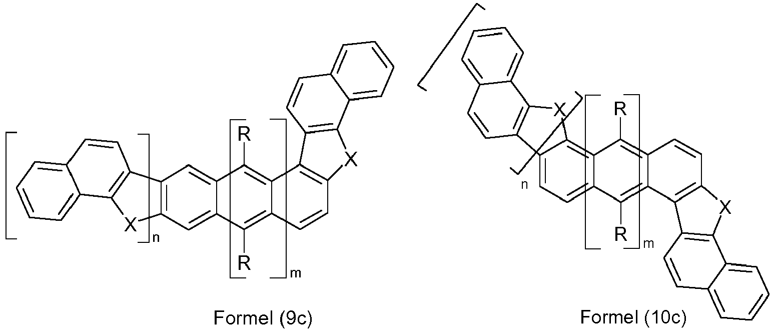 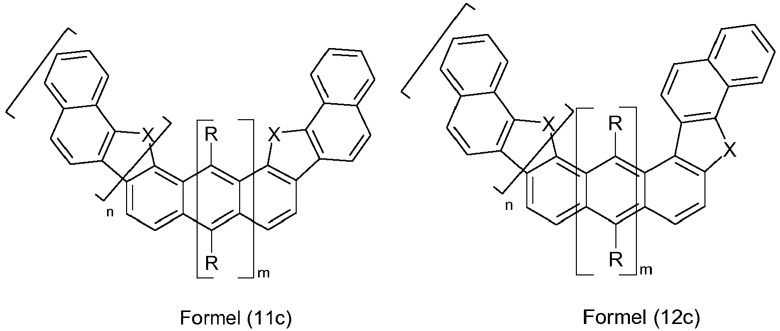 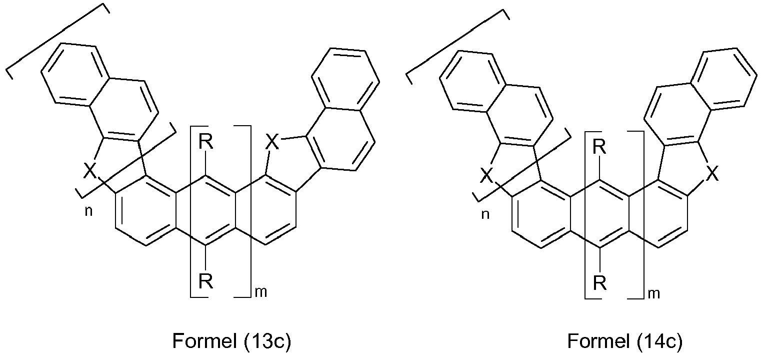 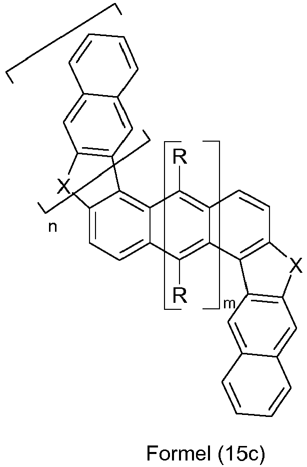 wobei die Symbole und Indizes die in Anspruch 1 aufgeführten Bedeutungen haben.

5. Verfahren zur Herstellung von Verbindungen nach einem oder mehreren der Ansprüche 1 bis 4 aus einem Dihalogenanthrachinon, umfassend die Reaktionsschritte:
a) Addition einer Arylmetallverbindung,
b) Reduktion zum Anthracen,
c) Kupplung mit einem funktionalisierten Aromaten, gegenenfalls nach Umwandlung der Halogengruppe in ein Boronsäurederivat, und
d) Ringschluss, bevorzugt unter sauren Bedingungen.

6. Dimere, Trimere, Tetramere, Pentamere, Oligomere, Polymere oder Dendrimere enthaltend eine oder mehrere Verbindungen nach einem oder mehreren der Ansprüche 1 bis 4, wobei ein oder mehrere Reste R, R¹ oder R² Bindungen zwischen den Verbindungen gemäß Formel (1) bzw. (2) im Dimer, Trimer, Tetramer bzw. Pentamer bzw. Bindungen der Verbindung gemäß Formel (1) bzw. (2) zum Polymer, Oligomer oder Dendrimer darstellen.

7. Verwendung von Verbindungen nach einem oder mehreren der Ansprüche 1 bis 4 oder 6 in elektronischen Vorrichtungen, insbesondere in organischen Elektrolumineszenzvorrichtungen (OLEDs, PLEDs), organischen Feld-Effekt-Transistoren (O-FETs), organischen Dünnfilmtransistoren (O-TFTs), organischen lichtemittierenden Transistoren (O-LETs), organischen integrierten Schaltungen (O-ICs), organischen Solarzellen (O-SCs), organischen Feld-Quench-Devices (O-FQDs), lichtemittierenden elektrochemischen Zellen (LECs), organischen Laserdioden (O-Laser) oder organischen Photorezeptoren.

8. Elektronische Vorrichtung, insbesondere ausgewählt aus organischen Elektrolumineszenzvorrichtungen (OLEDs, PLEDs), organischen Feld-Effekt-Transistoren (O-FETs), organischen Dünnfilmtransistoren (O-TFTs), organischen lichtemittierenden Transistoren (O-LETs), organischen integrierten Schaltungen (O-ICs), organischen Solarzellen (O-SCs), organischen Feld-Quench-Devices (O-FQDs), lichtemittierenden elektrochemischen Zellen (LECs), organischen Laserdioden (O-Laser) und organischen Photorezeptoren, enthaltend mindestens eine Verbindung nach einem oder mehreren der Ansprüche 1 bis 4 oder 6.

9. Organische Elektrolumineszenzvorrichtung nach Anspruch 8, **dadurch gekennzeichnet, dass** diese außer Anode, Kathode und der emittierenden Schicht noch weitere Schichten enthält, ausgewählt aus aus jeweils einer oder mehreren Lochinjektionsschichten, Lochtransportschichten, Lochblockierschichten, Elektronentransportschichten, Elektroneninjektionsschichten, Elektronenblockierschichten, Charge-Generation Layers und/oder weiteren emittierenden Schichten, wobei nicht notwendigerweise jede dieser Schichten vorhanden sein muss.

10. Organische Elektrolumineszenzvorrichtung nach Anspruch 8 oder 9, **dadurch gekennzeichnet, dass** die Verbindung nach einem oder mehreren der Ansprüche 1 bis 4 als Hostmaterial für fluoreszierende Dotanden, als fluoreszierender Dotand, als Lochtransportmaterial, als Lochinjektionsmaterial oder als Elektronentransportmaterial eingesetzt wird.

## Claims

1. Compounds of the formulae (1) and (2), 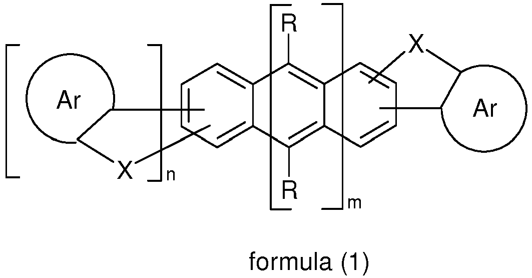 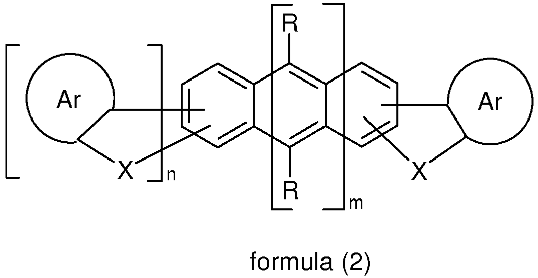 where one or more unsubstituted carbon atoms in the anthracene unit may also be replaced by nitrogen; furthermore, the following applies to the symbols and indices used:
X is on each occurrence, identically or differently, a divalent bridge selected from C(R¹)₂;
Ar is on each occurrence, identically or differently, an aryl group having 6 to 10 C atoms or a heteroaryl group having 4 to 9 C atoms;
R¹ is on each occurrence, identically or differently, methyl, ethyl, isopropyl, tert-butyl, where one H atom or a plurality of H atoms may be replaced by F, or an aryl group having 6 to 14 C atoms, which may in each case be substituted by one or more radicals R²; two or more adjacent substituents R¹ may also form a mono-or polycyclic, aliphatic or aromatic ring system with one another;
R is on each occurrence, identically or differently, an aromatic or heteroaromatic ring system having 5 to 14 aromatic ring atoms, which may in each case be substituted by one or more radicals R²;
R² is on each occurrence, identically or differently, H, D, F, Cl, methyl, ethyl, n-propyl, i-propyl, n-butyl, i-butyl, s-butyl, t-butyl, where one or more H atoms may be replaced by F, Cl or CN;
m is 1;
n is 0 or 1;
**characterised in that** the groups Ar are unsubstituted.

2. Compounds according to Claim 1 of the formulae (3) to (15), 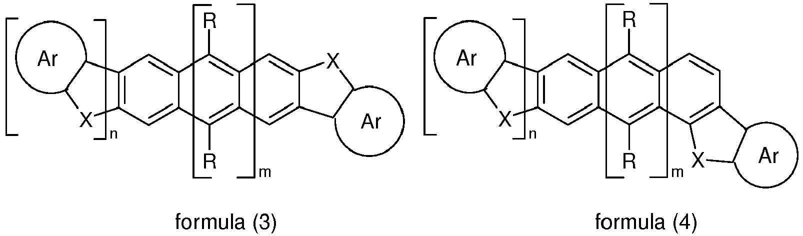 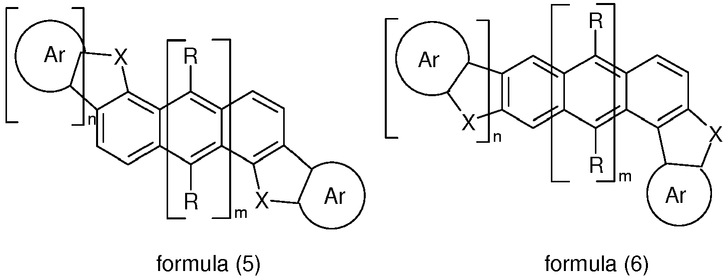 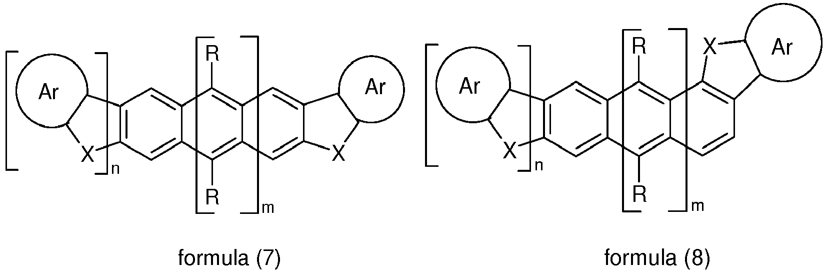 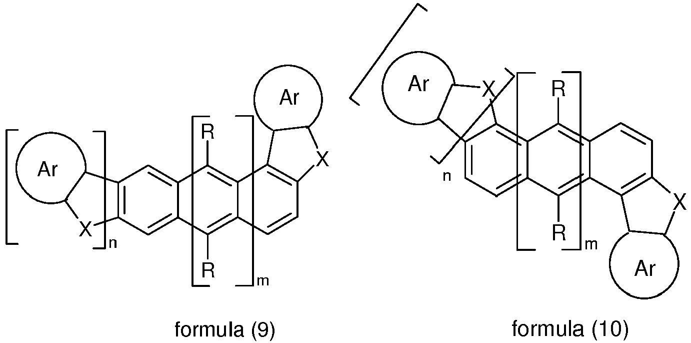 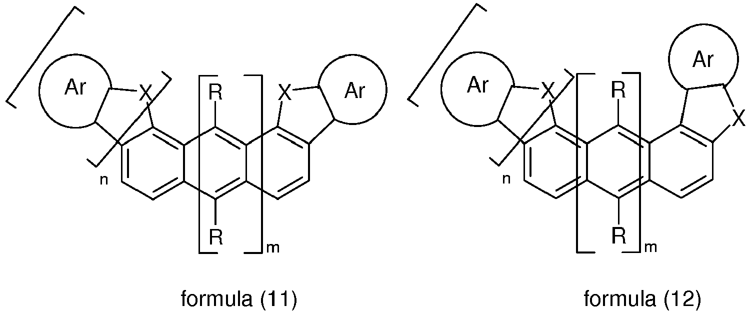 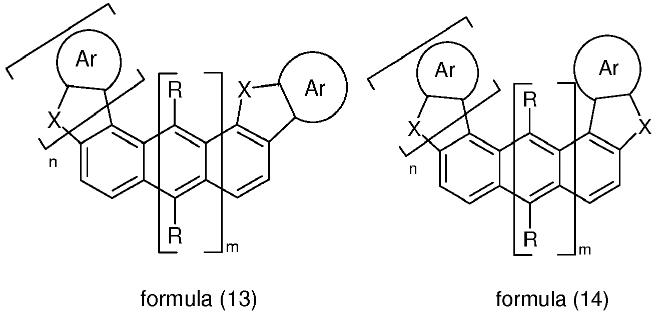 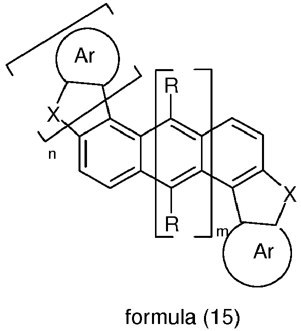 where one or more unsubstituted carbon atoms in the anthracene unit may also be replaced by nitrogen and where the symbols and indices have the meanings indicated in Claim 1.

3. Compounds according to Claim 1 or 2, **characterised in that** the symbol Ar stands, identically or differently on each occurrence, for benzene, naphthalene, thiophene, pyrrole, furan, pyridine, pyrimidine, pyrazine, pyridazine, quinoline, quinoxaline, benzothiophene, indole or benzofuran.

4. Compounds according to one or more of Claims 1 to 3 of the formulae (3a) to (15a), (3b) to (15b) and (3c) to (15c), 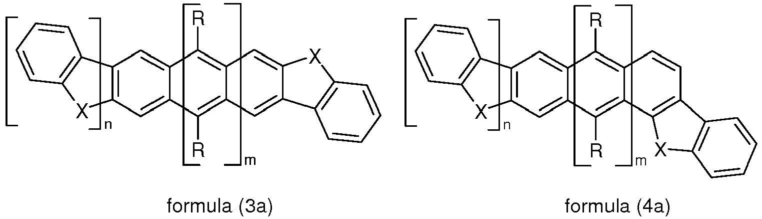 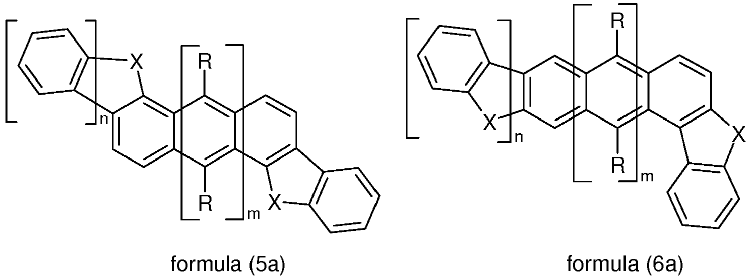 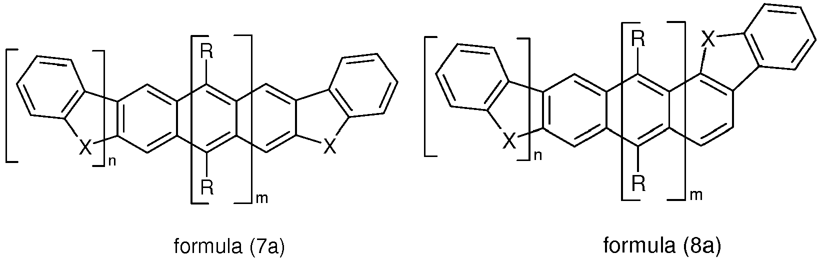 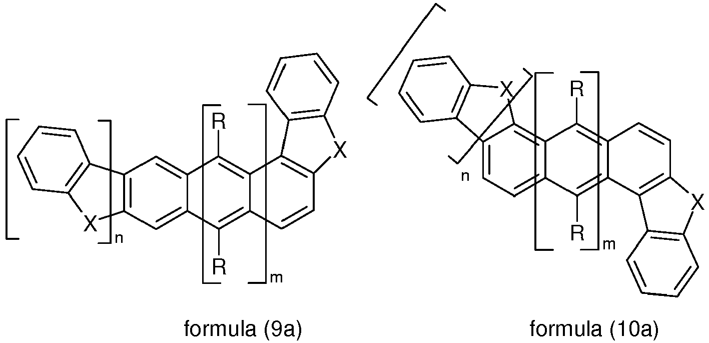 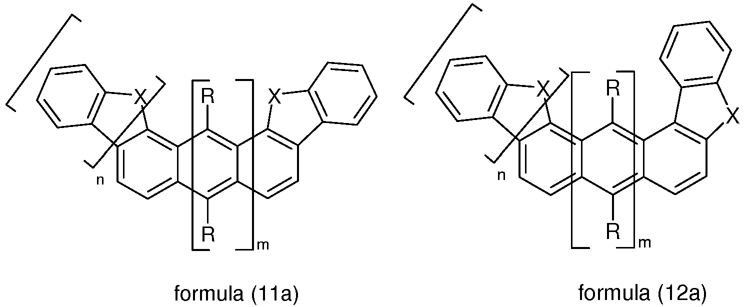 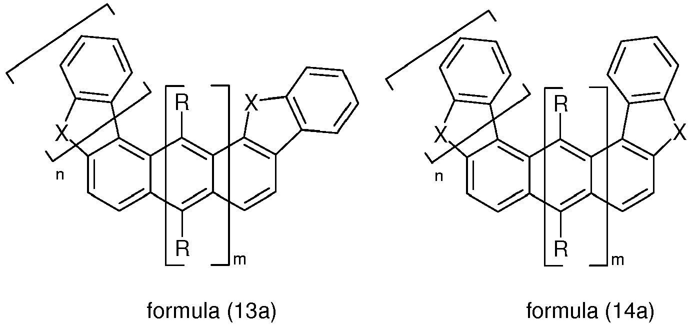 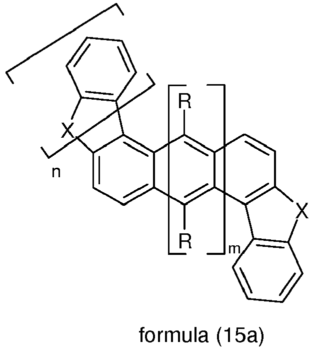 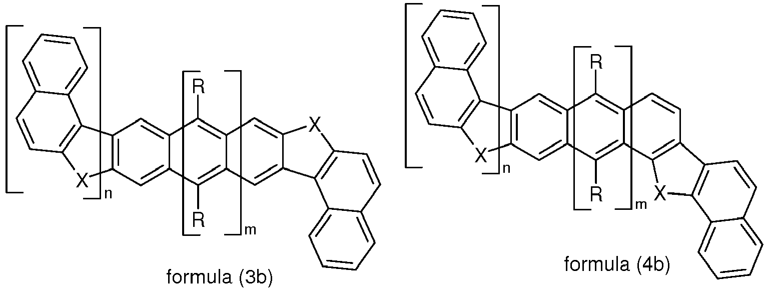 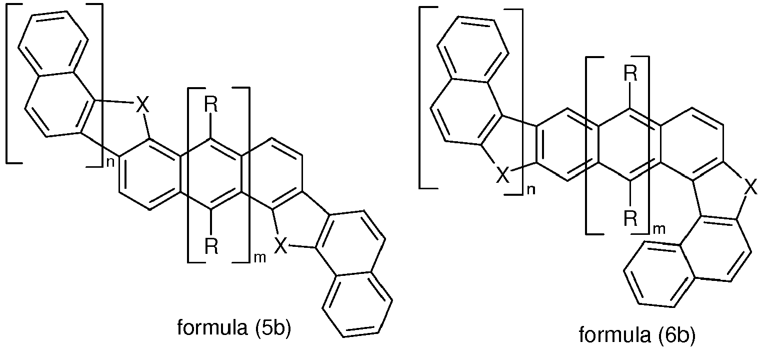 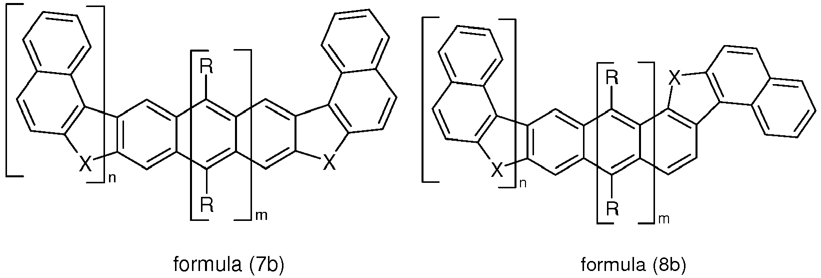 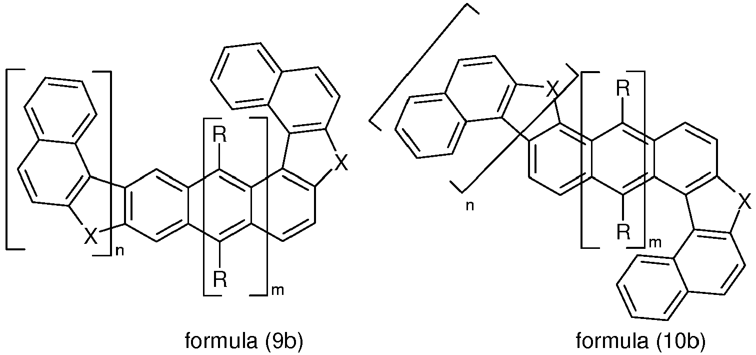 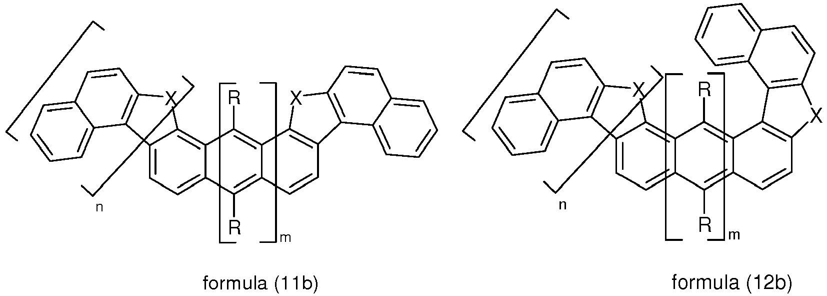 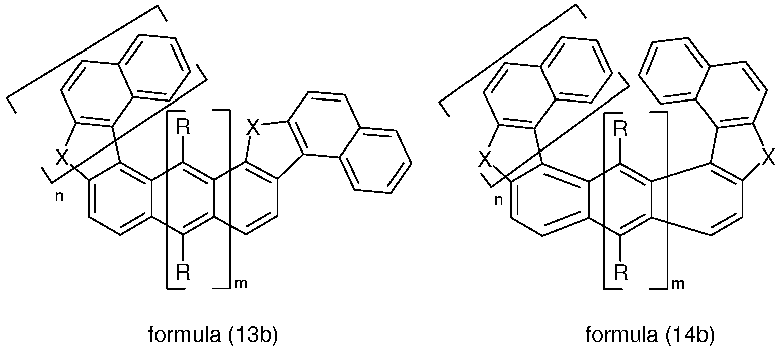 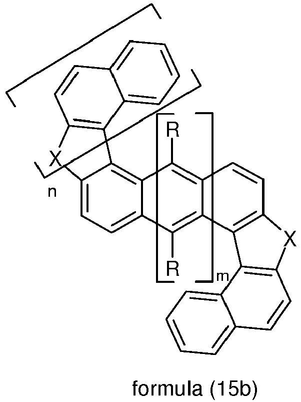 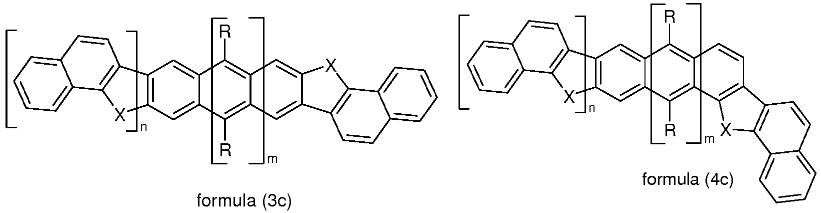 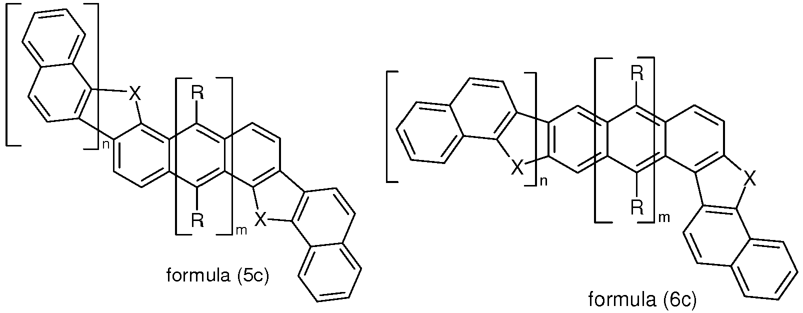 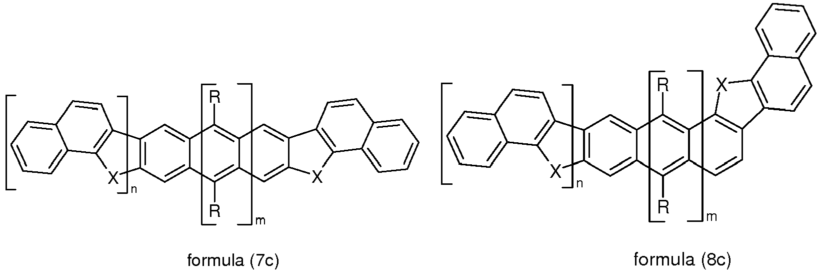 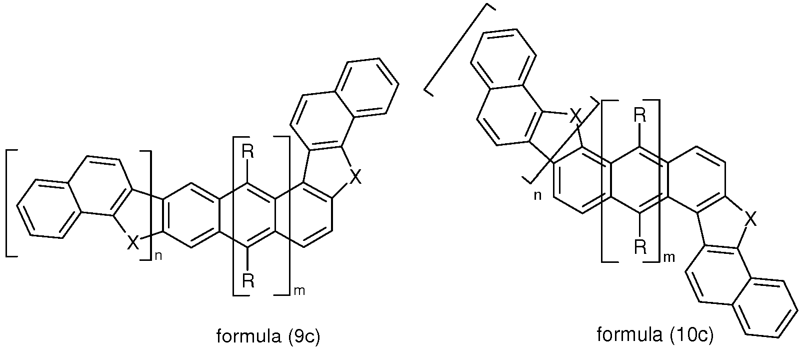 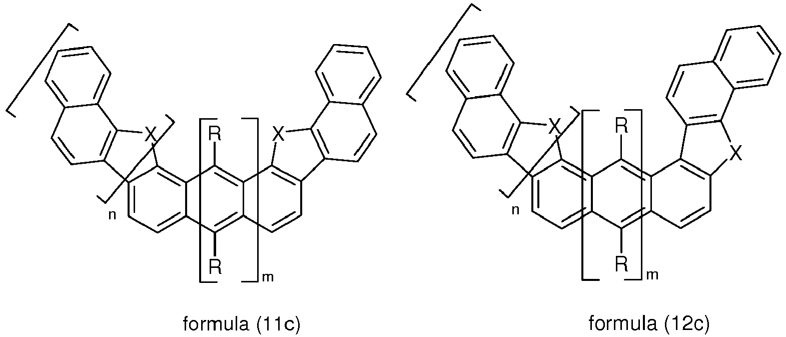 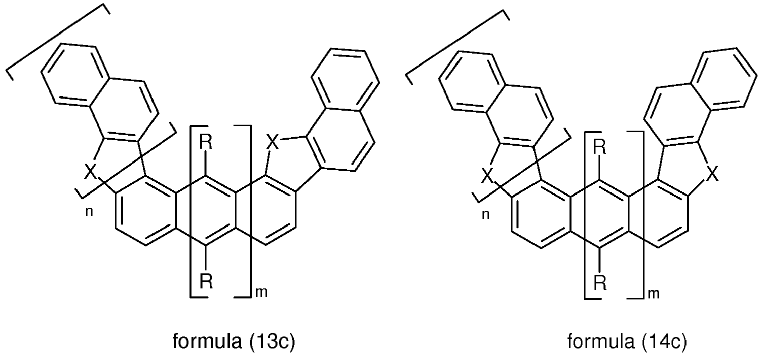 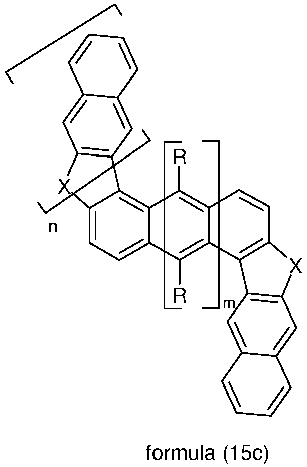 where the symbols and indices have the meanings indicated in Claim 1.

5. Process for the preparation of compounds according to one or more of Claims 1 to 4 from a dihaloanthraquinone, comprising the reaction steps:
a) addition reaction of an arylmetal compound,
b) reduction to the anthracene,
c) coupling to a functionalised aromatic compound, optionally after conversion of the halogen group into a boronic acid derivative, and
d) ring closure, preferably under acidic conditions.

6. Dimers, trimers, tetramers, pentamers, oligomers, polymers or dendri-mers comprising one or more compounds according to one or more of Claims 1 to 4, where one or more radicals R, R¹ or R² represent bonds between the compounds of the formula (1) or (2) in the dimer, trimer, tetramer or pentamer or bonds from the compound of the formula (1) or (2) to the polymer, oligomer or dendrimer.

7. Use of compounds according to one or more of Claims 1 to 4 or 6 in electronic devices, in particular in organic electroluminescent devices (OLEDs, PLEDs), organic field-effect transistors (O-FETs), organic thin-film transistors (O-TFTs), organic light-emitting transistors (O-LETs), organic integrated circuits (O-ICs), organic solar cells (O-SCs), organic field-quench devices (O-FQDs), light-emitting electrochemical cells (LECs), organic laser diodes (O-lasers) or organic photoreceptors.

8. Electronic device, in particular selected from organic electroluminescent devices (OLEDs, PLEDs), organic field-effect transistors (O-FETs), organic thin-film transistors (O-TFTs), organic light-emitting transistors (O-LETs), organic integrated circuits (O-ICs), organic solar cells (O-SCs), organic field-quench devices (O-FQDs), light-emitting electrochemical cells (LECs), organic laser diodes (O-lasers) and organic photoreceptors, comprising at least one compound according to one or more of Claims 1 to 4 or 6.

9. Organic electroluminescent device according to Claim 8, **characterised in that**, apart from anode, cathode and the emitting layer, it also comprises further layers selected from in each case one or more hole-injection layers, hole-transport layers, hole-blocking layers, electron-transport layers, electron-injection layers, electron-blocking layers, charge-generation layers and/or further emitting layers, where each of these layers does not necessarily have to be present.

10. Organic electroluminescent device according to Claim 8 or 9, **characterised in that** the compound according to one or more of Claims 1 to 4 is employed as host material for fluorescent dopants, as fluorescent dopant, as hole-transport material, as hole-injection material or as electron-transport material.

## Revendications

1. Composés des formules (1) et (2) : 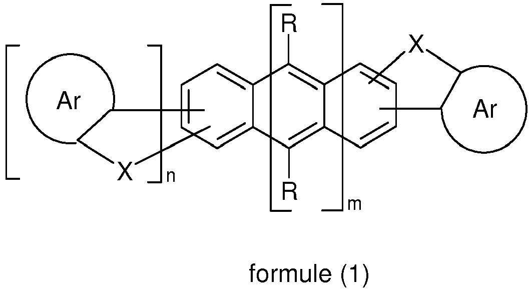 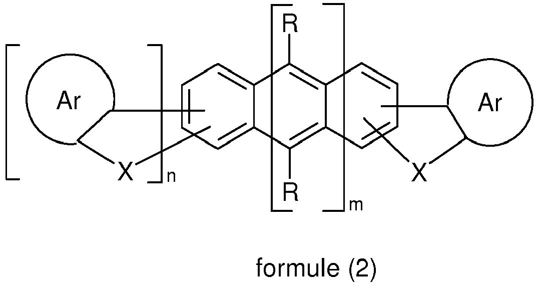 dans lesquelles un ou plusieurs atome(s) de carbone non substitué(s) dans l'unité anthracène peut/peuvent également être remplacé(s) par azote ; en outre, ce qui suit s'applique aux symboles et indices qui sont utilisés :
X est pour chaque occurrence, de manière identique ou différente, un pont divalent qui est sélectionné parmi C(R¹)₂ ;
Ar est pour chaque occurrence, de manière identique ou différente, un groupe aryle qui comporte de 6 à 10 atomes de C ou un groupe hétéroaryle qui comporte de 4 à 9 atomes de C ;
R¹ est pour chaque occurrence, de manière identique ou différente, méthyle, éthyle, isopropyle, tert-butyle, où un atome de H ou une pluralité d'atomes de H peut/peuvent être remplacé(s) par F, ou un groupe aryle qui comporte de 6 à 14 atomes de C, le-quel peut dans chaque cas être substitué par un radical ou par plusieurs radicaux R² ; deux substituants R¹ adjacents ou plus peuvent également former un système de cycle aliphatique ou aromatique mono- ou polycyclique l'un avec l'autre ou les uns avec les autres ;
R est pour chaque occurrence, de manière identique ou différente, un système de cycle aromatique ou hétéroaromatique qui com-porte de 5 à 14 atomes de cycle aromatique, lequel peut dans chaque cas être substitué par un radical ou par plusieurs radi-caux R² ;
R² est pour chaque occurrence, de manière identique ou différente, H, D, F, Cl, méthyle, éthyle, n-propyle, i-propyle, n-butyle, i-butyle, s-butyle, t-butyle, où un ou plusieurs atome(s) de H peut/peuvent être remplacé(s) par F, Cl ou CN ;
m est 1 ;
n est 0 ou 1 ;
**caractérisés en ce que** les groupes Ar sont non substitués.

2. Composés selon la revendication 1 des formules (3) à (15) : 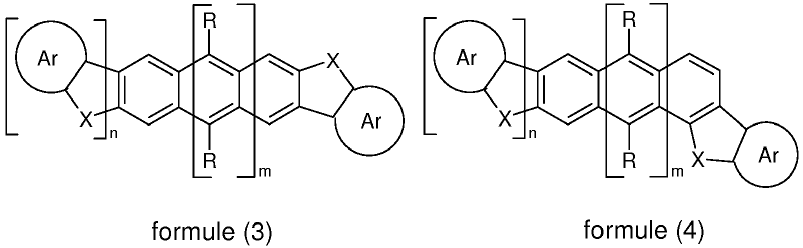 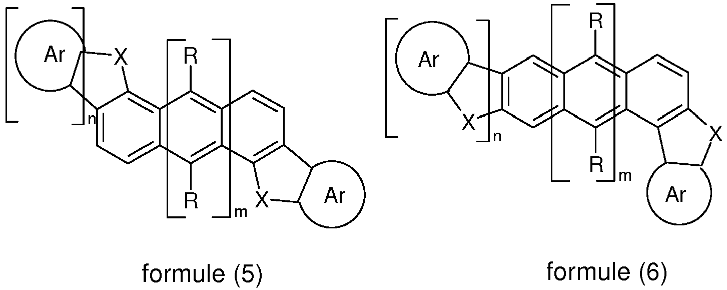 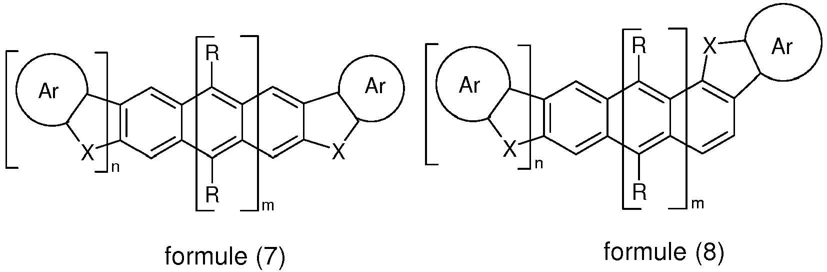 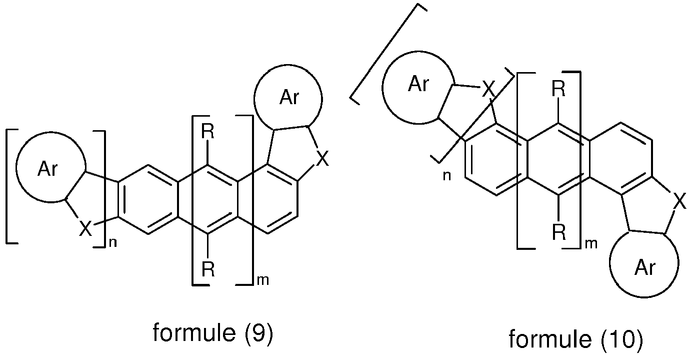 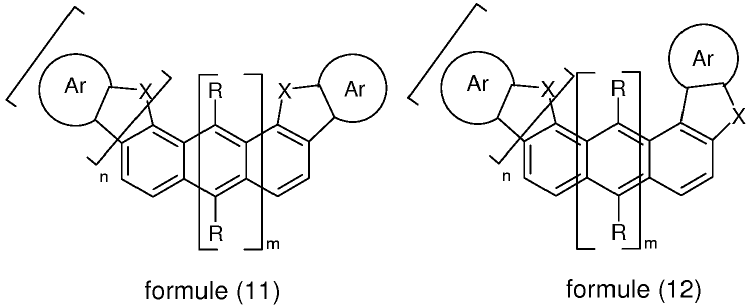 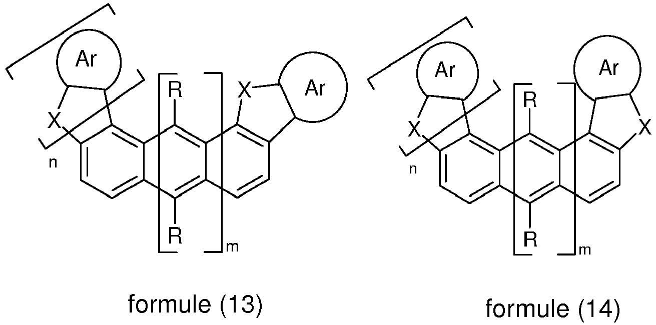 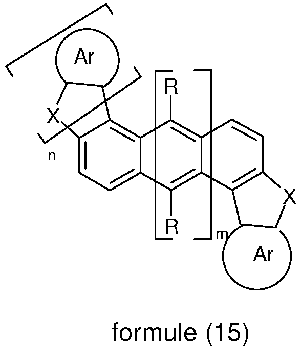 dans lesquelles un ou plusieurs atome(s) de carbone non substitué(s) dans l'unité anthracène peut/peuvent également être remplacé(s) par azote et dans lesquelles les symboles et indices présentent les significations qui ont été indiquées selon la revendication 1.

3. Composés selon la revendication 1 ou 2, **caractérisés en ce que** le symbole Ar représente, de manière identique ou différente pour chaque occurrence, benzène, naphtalène, thiophène, pyrrole, furane, pyridine, pyrimidine, pyrazine, pyridazine, quinoline, quinoxaline, benzothiophène, indole ou benzofurane.

4. Composés selon une ou plusieurs des revendications 1 à 3 des formules (3a) à (15a), (3b) à (15b) et (3c) à (15c) : 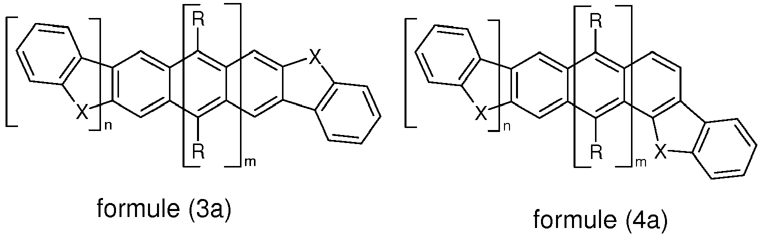 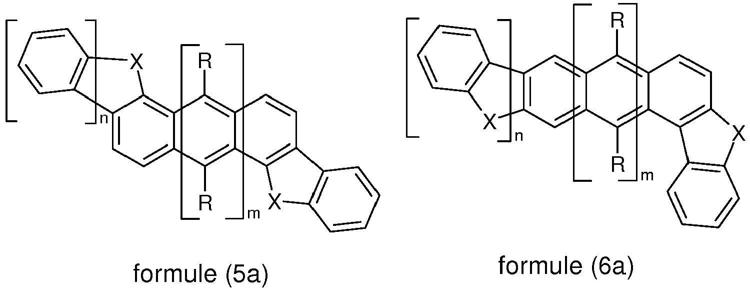 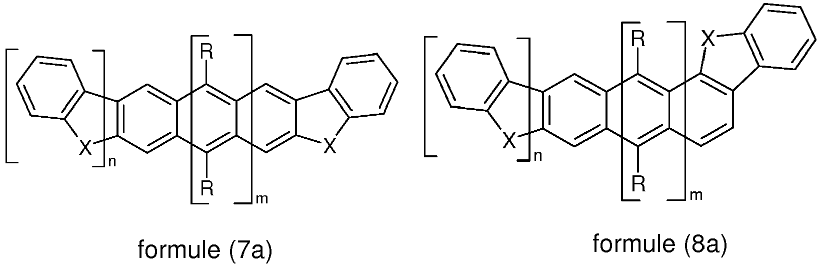 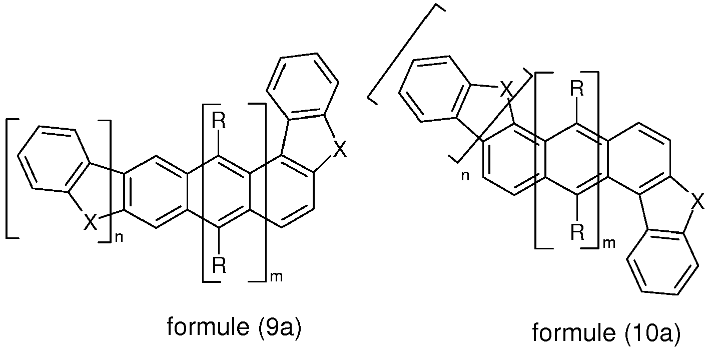 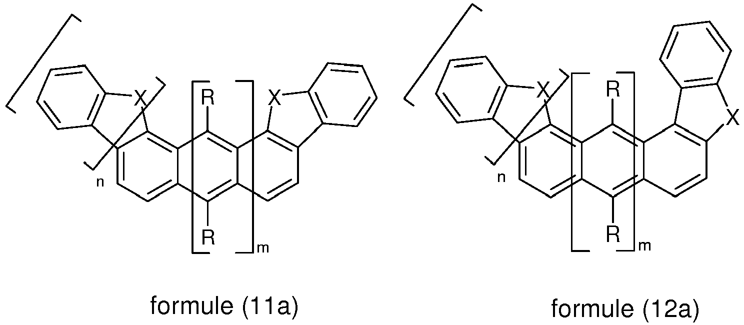 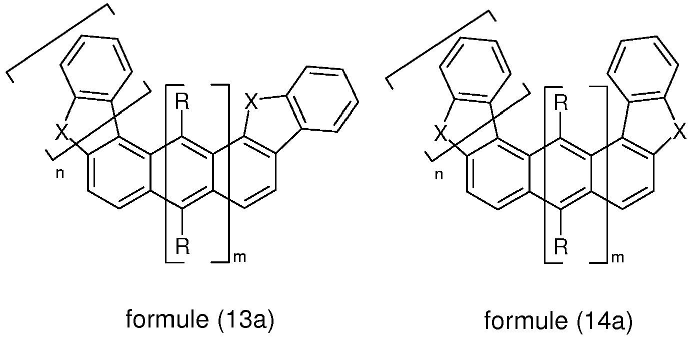 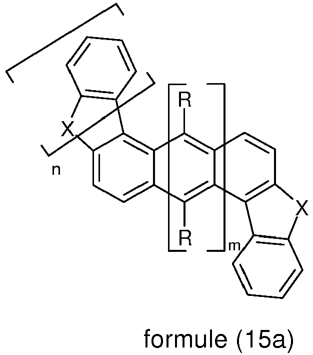 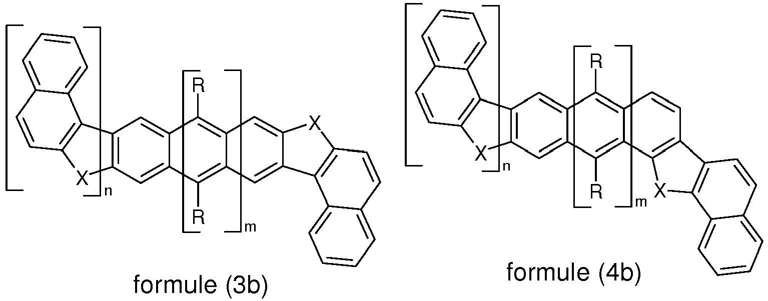 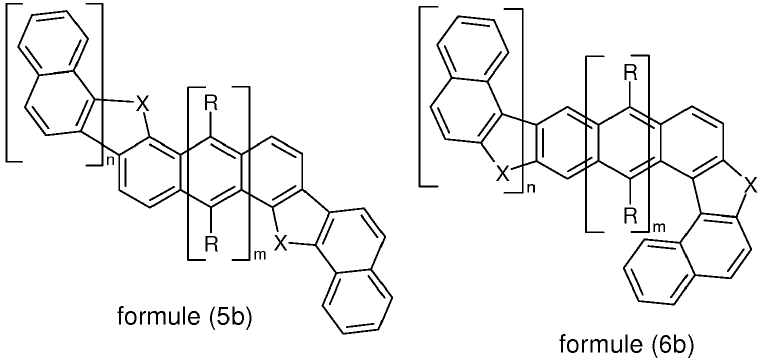 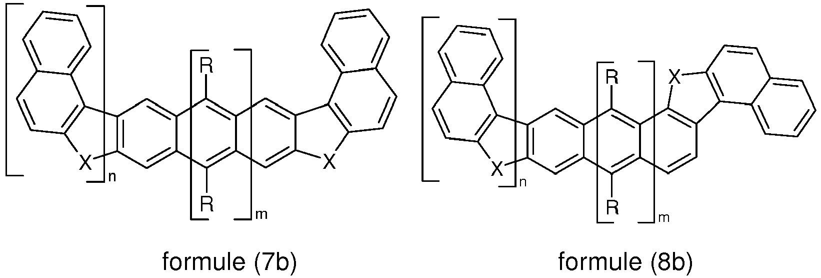 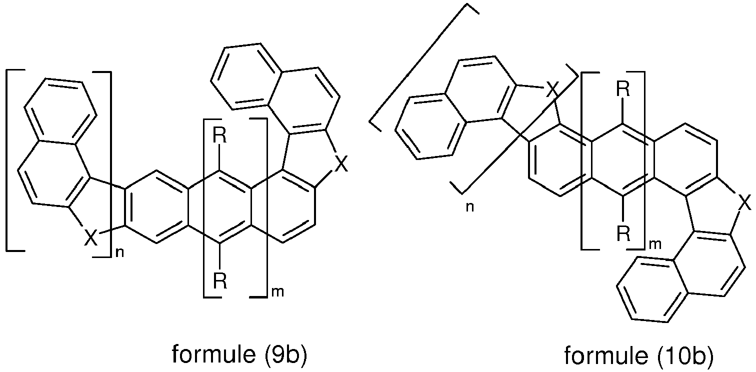 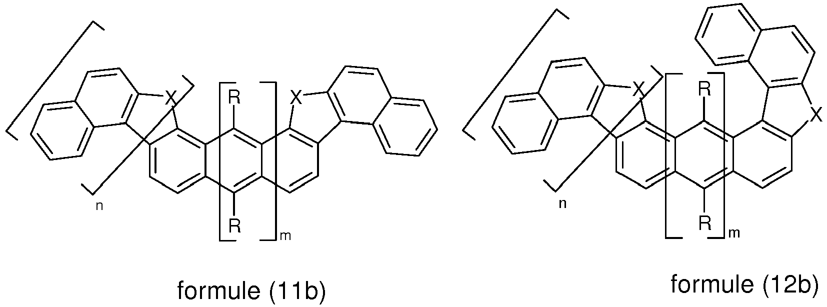 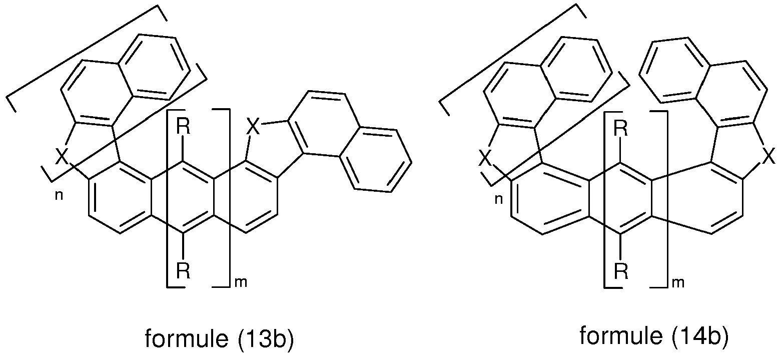 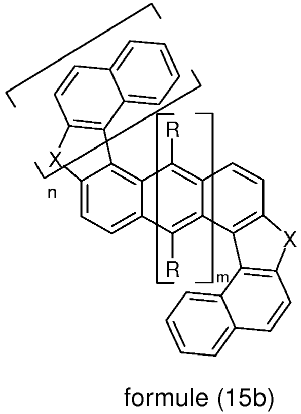 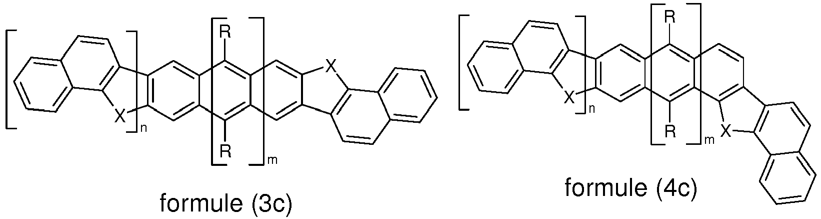 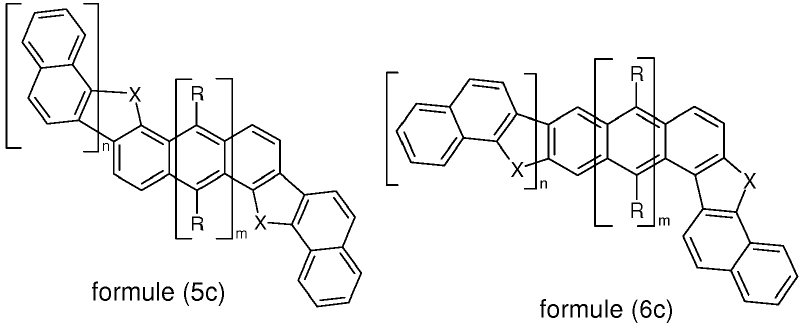 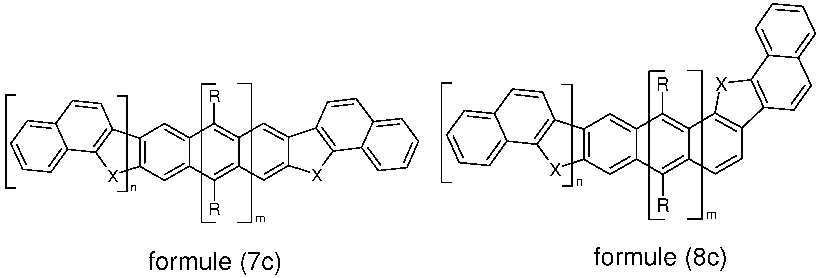 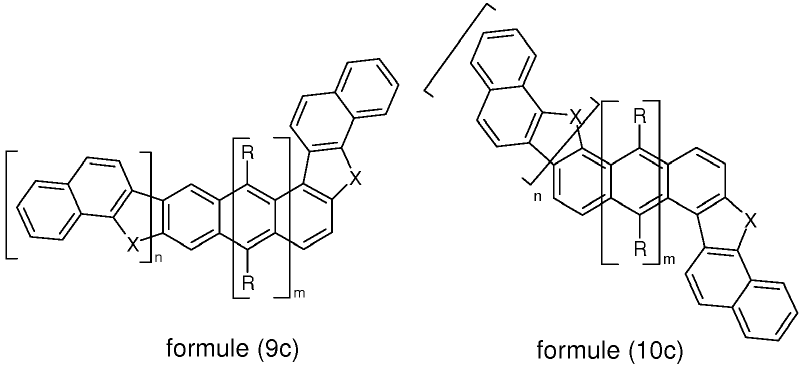 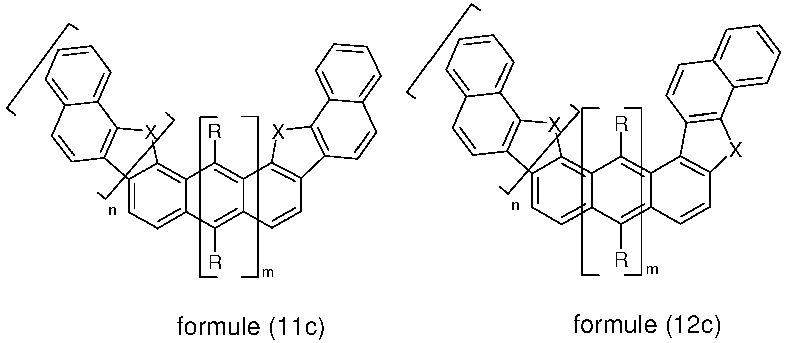 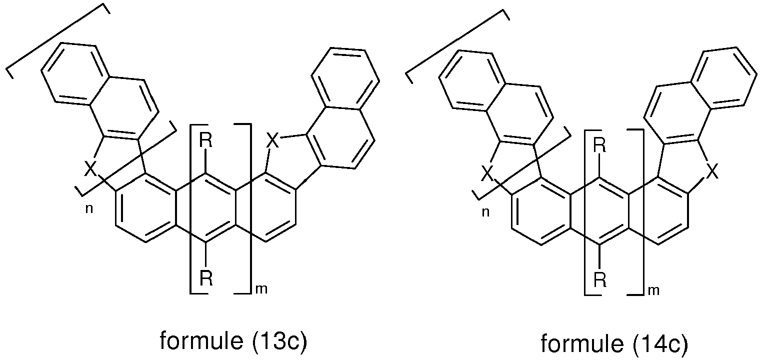 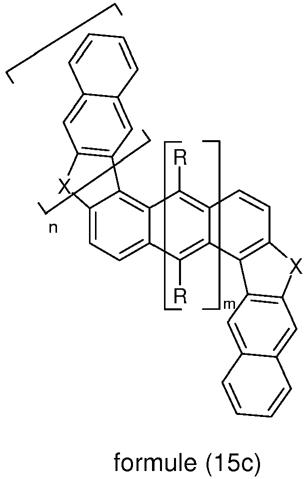 dans lesquelles les symboles et indices présentent les significations qui ont été indiquées selon la revendication 1.

5. Procédé pour la préparation de composés selon une ou plusieurs des revendications 1 à 4 à partir d'un dihaloanthraquinone, comprenant les étapes de réaction qui suivent :
a) une réaction d'addition d'un composé arylmétal ;
b) une réduction selon l'anthracène ;
c) un couplage sur un composé aromatique fonctionnarisé, en option après conversion du groupe halogène selon un dérivé d'acide boron-ique ; et
d) une fermeture de cycle, de préférence sous des conditions acides.

6. Dimères, trimères, tétramères, pentamères, oligomères, polymères ou dendrimères comprenant un ou plusieurs composé(s) selon une ou plusieurs des revendications 1 à 4, où un radical ou plusieurs radicaux R, R¹ ou R² représente/représentent des liaisons entre les composés de la formule (1) ou (2) dans le dimère, le trimère, le tétramère ou le pentamère ou des liaisons depuis le composé de la formule (1) ou (2) sur le polymère, l'oligomère ou le dendrimère.

7. Utilisation de composés selon une ou plusieurs des revendications 1 à 4 ou 6 dans des dispositifs électroniques, en particulier dans des dispositifs électroluminescents organiques (OLED, PLED), des transistors à effet de champ organiques (O-FET), des transistors à film mince organiques (O-TFT), des transistors à émission de lumière organiques (O-LET), des circuits intégrés organiques (O-IC), des cellules solaires organiques (O-SC), des dispositifs à extinction de champ organiques (O-FQD), des cellules électrochimiques à émission de lumière (LEC), des diodes laser organiques (O-laser) ou des photorécepteurs organiques.

8. Dispositif électronique, en particulier sélectionné parmi les dispositifs électroluminescents organiques (OLED, PLED), les transistors à effet de champ organiques (O-FET), les transistors à film mince organiques (O-TFT), les transistors à émission de lumière organiques (O-LET), les circuits intégrés organiques (O-IC), les cellules solaires organiques (O-SC), les dispositifs à extinction de champ organiques (O-FQD), les cellules électrochimiques à émission de lumière (LEC), les diodes laser organiques (O-laser) et les photorécepteurs organiques, comprenant au moins un composé selon une ou plusieurs des revendications 1 à 4 ou 6.

9. Dispositif électroluminescent organique selon la revendication 8, **caractérisé en ce que**, indépendamment d'une anode, d'une cathode et de la couche d'émission, il comprend également d'autres couches qui sont sélectionnées parmi, dans chaque cas, une ou plusieurs couches d'injection de trous, une ou plusieurs couches de transport de trous, une ou plusieurs couches de blocage de trous, une ou plusieurs couches de transport d'électrons, une ou plusieurs couches d'injection d'électrons, une ou plusieurs couches de blocage d'électrons, une ou plusieurs couches de génération de charges et/ou d'autres couches d'émission, où chacune de ces couches ne doit pas nécessairement être présente.

10. Dispositif électroluminescent organique selon la revendication 8 ou 9, **caractérisé en ce que** le composé selon une ou plusieurs des revendications 1 à 4 est utilisé en tant que matériau hôte pour des dopants fluorescents, en tant que dopant fluorescent, en tant que matériau de transport de trous, en tant que matériau d'injection de trous ou en tant que matériau de transport d'électrons.
